(19) Europäisches Patentamt
European Patent Office
Office européen des brevets

(11) **EP 4 358 838 B1**

(12) **EUROPEAN PATENT SPECIFICATION**

(45) Date of publication and mention
of the grant of the patent:
**01.10.2025  Bulletin 2025/40**

(21) Application number: **23761406.0**

(22) Date of filing: **04.08.2023**

(51) International Patent Classification (IPC):
**A61B 5/103** *(2006.01)*        **A61B 5/11** *(2006.01)*
**A61B 5/00** *(2006.01)*

(52) Cooperative Patent Classification (CPC):
**A61B 5/1036; A61B 5/1118; A61B 5/681**

(86) International application number:
**PCT/US2023/071719**

(87) International publication number:
**WO 2024/031079 (08.02.2024 Gazette 2024/06)**

(54) **MUSCULOSKELETAL STRAIN**

MUSKEL-SKELETT-BELASTUNG

TENSION MUSCULO-SQUELETTIQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:   **04.08.2022   US 202263395244 P**

(43) Date of publication of application:
**01.05.2024   Bulletin 2024/18**

(73) Proprietor: **Whoop, Inc.
Boston, MA 02215 (US)**

(72) Inventors:
• **BABAKESHIZADEH, Vahid
Boston MA 02215 (US)**
• **CAPODILUPO, Emily Rachel
Boston MA 02215 (US)**
• **DIRANEYYA, Mohsen Mu'tasem Ma'moun
Boston MA 02215 (US)**
• **CHAPMAN, Christopher John
Boston MA 02215 (US)**

(74) Representative: **Black, Diego
Withers & Rogers LLP
2 London Bridge
London SE1 9RA (GB)**

(56) References cited:
WO-A1-2015/048884      WO-A1-2021/023357
US-A1- 2018 133 551      US-A1- 2021 128 979

**Description**

CROSS-REFERENCE TO RELATED APPLICATIONS

This application claims priority to U.S. Provisional Patent App. No. 63/395,244 filed on August 4, 2022.

TECHNICAL FIELD

This disclosure relates to physiological monitoring systems, and more specifically to techniques for quantitatively tracking musculoskeletal strain.

BACKGROUND

Wearable physiological monitors can provide a wealth of physiological data from a wearer. However, muscular strain incurred during strength training can be difficult to characterize using conventional fitness metrics such as heart rate or heart rate variability. There remains a need for improved methods and systems for monitoring musculoskeletal strain, and for using quantitatively measured strain to provide coaching recommendations and the like.
WO 2021/023357 A1 describes a system for monitoring and evaluating physical movements of a user, the system comprising: a detection unit configured to receive a signal obtained by a sensor device representative of a sensed bodily activity, such as a muscle activity, calculate a load index value and communicate the value of the load index value.
WO 2015/048884 A1 describes a system and method for tracking and analyzing exercises and managing training regimens.
US 2021/128979 A1 describes a wearable device configured to sense movement and generate motion sensor data from the wearable device and an exercise device in communication with the wearable device.
US 2018/133551 A1 describes a system and method for generating a set of training metrics that comprises at least one training metric.

SUMMARY

A physiological monitor uses patterns of motion during strength training activity, e.g., as detected by a wearable monitor, to evaluate a degree of muscular, musculoskeletal, and/or biomechanical strain experienced by a user while engaged in strength training. The resulting strain may advantageously be quantified and used to provide coaching recommendations, update daily strain metrics, and take other responsive actions.
In an aspect, a computer program product disclosed herein may include computer executable code embodied in a non-transitory computer readable medium that, when executing on one or more computing devices, causes the one or more computing devices to perform the steps of: receiving raw motion data from one or more motion sensors of a wearable fitness monitor worn by a user during a strength training activity including a set of one or more repetitions, the raw motion data including angular rotation data from a plurality of gyroscopes and linear acceleration data from a plurality of accelerometers; fusing the raw motion data from the one or more motion sensors to mitigate gravitational artifacts, thereby providing motion data including three-axis acceleration data; identifying a type of the strength training activity; determining a quantity of the repetitions in the set based on variations in a magnitude of the three-axis acceleration data; for each one of the repetitions, calculating a raw intensity score indicative of musculoskeletal movement based on changes in the three-axis acceleration data; for each one of the repetitions, determining a maximum intensity for performing the strength training activity by the user, the maximum intensity indicative of a capacity of the user to perform the strength training activity based on an exercise history for the user; estimating a maximum volume for the user and the strength training activity based on a history of user performance with the strength training activity, where the maximum volume is indicative of an upper threshold for injury-free repetitions of the strength training activity by the user; calculating an effective load for the user during the strength training activity, the effective load indicative of a relative portion of the maximum volume exerted by the user during the strength training activity based on one or more load parameters including at least a body weight of the user and an added weight for the strength training activity; calculating a per repetition musculoskeletal strain for each one of the repetitions as a product of a first ratio of the effective load to the maximum volume and a second ratio of the raw intensity score to the maximum intensity; summing the per repetition musculoskeletal strain for all of the repetitions in the set to provide a musculoskeletal strain score for the strength training activity; and displaying the musculoskeletal strain score for the strength training activity to the user.
Implementations may include one or more of the following features. The computer program product may include code that causes the one or more computing devices to perform the step of generating a coaching recommendation to the user based on the musculoskeletal strain score. The coaching recommendation may be based at least in part on a fitness goal for the user. Calculating the raw intensity score for one of the repetitions may include calculating a mean of a number of instantaneous intensity measurements for the one of the repetitions. One or more of the number of instantaneous intensity measurements may be calculated based on a mean of a ratio of discretely measured changes in a current acceleration to the current acceleration. One or more of the number of instantaneous intensity measurements may be calculated based on a ratio of a first mean of a current change in acceleration to a second mean of a current acceleration. The computer program product may include code that causes the one or more computing devices to perform the step of creating a load-repetition profile for the user based on a history of the strength training activity by the user, the load-repetition profile indicating a capacity for repetitions by the user at one or more loads during the strength training activity. The computer program product may include code that causes the one or more computing devices to perform the step of adding a row to the load-repetition profile when the user performs the strength training activity at a new load that is not included in the one or more loads in the load-repetition profile. The computer program product may include code that causes the one or more computing devices to perform the step of updating the load-repetition profile when the user exceeds a number of repetitions for one of the loads in the load-repetition profile. The computer program product may include code that receives a user input specifying the type of the strength training activity. The computer program product may include code that causes the one or more computing devices to perform the step of identifying the type of the strength training activity based on the raw motion data. Implementations of the described techniques may include hardware, a method or process, computer software on a computer-accessible medium, and a system.

In an example, a method disclosed herein may include: receiving motion data from one or more motion sensors of a wearable monitor worn by a user during a strength training activity including a set of one or more repetitions; identifying a type of the strength training activity; determining a quantity of the repetitions in the set; for each one of the repetitions, calculating a raw intensity score indicative of musculoskeletal movement based on features of the motion data, and scaling the raw intensity score relative to a maximum intensity for performing the strength training activity by the user to obtain a per repetition user intensity score, the maximum intensity indicative of a capacity of the user to perform the strength training activity based on an exercise history for the user; for each one of the repetitions, calculating an individualization scale based on a ratio of an effective load for the user during the strength training activity and a predetermined load threshold for the user performing the strength training activity; calculating a per repetition musculoskeletal strain for each one of the repetitions as a product of the per repetition user intensity score and the individualization scale; calculating a musculoskeletal strain score for the strength training activity by summing the per repetition musculoskeletal strain for all of the repetitions in the set; and taking an action based on the musculoskeletal strain score. Other embodiments of this aspect may also or instead include a computer program product comprising computer executable code embodied in a non-transitory computer readable medium that, when executing on one or more computing devices, causes the one or more computing devices to perform one or more of the aforementioned steps. Other embodiments of this example may also or instead include a system having a wearable fitness monitor including one or more motion sensors and one or more processors configured to calculate a user-specific musculoskeletal strain score for a user of the wearable fitness monitor by performing one or more of the aforementioned steps.

Implementations may include one or more of the following features. Determining the quantity of the repetitions in the set may include determining the quantity based on motion data. Determining the quantity of the repetitions in the set may include determining the quantity based on user input. The action may include refining a daily strain calculation for the user based on the musculoskeletal strain score. The action may include generating a coaching recommendation for the user. The method may include displaying the coaching recommendation to the user. The coaching recommendation may be a real time coaching recommendation. The coaching recommendation may relate to a subsequent exercise activity by the user. The method may include automatically identifying the type of the strength training activity based on the motion data. The method may include calculating a plurality of musculoskeletal strain scores for each of a plurality of types of strength training activity in an exercise routine. The method may include calculating the effective load based on a user input of a body weight for the user. The method may include calculating the effective load based on a user input of an added weight for the strength training activity. The motion data may include raw motion data from a three-axis gyroscope and a three-axis accelerometer fused to provide three-axis acceleration data for the repetitions with mitigated effects of acceleration due to gravity. The wearable monitor may include a wrist-worn photoplethysmography device. Receiving motion data may include receiving raw motion data from at least one gyroscope and at least one accelerometer of the wearable monitor. Calculating the musculoskeletal strain score may include calculating the musculoskeletal strain score on a personal computing device of the user coupled in a communicating relationship with the wearable monitor. Calculating the musculoskeletal strain score may include calculating the musculoskeletal strain score on a remote server coupled in a communicating relationship with the wearable monitor. The predetermined load threshold may be an estimated maximum volume indicative of an upper threshold for injury-free repetitions of the strength training activity by the user. Implementations of the described techniques may include hardware, a method or process, computer software on a computer-accessible medium, and a system.

In an example, a system disclosed herein may include a wearable fitness monitor including one or more motion sensors, and one or more processors configured to calculate a user-specific musculoskeletal strain score for a user of the wearable fitness monitor by performing the steps of: receiving motion data obtained from the one or more motion sensors during a strength training activity; identifying a type of the strength training activity; identifying a set of the strength training activity including one or more repetitions; for each one of the repetitions, calculating a raw intensity score indicative of musculoskeletal movement based on features of the motion data, and scaling the raw intensity score relative to a maximum intensity for performing the strength training activity by the user to obtain a per repetition user intensity score, the maximum intensity indicative of a capacity of the user to perform the strength training activity based on an exercise history for the user; for each one of the repetitions, calculating an individualization scale based on a ratio of an effective load for the user during the strength training activity and a predetermined load threshold for the user when performing the strength training activity; calculating a per repetition musculoskeletal strain for each one of the repetitions as a product of the per repetition user intensity score and the individualization scale; calculating a musculoskeletal strain score for the strength training activity by summing the per repetition musculoskeletal strain for all of the repetitions in the set; and taking an action based on the musculoskeletal strain score. Taking the action may include transmitting the user-specific musculoskeletal strain score to a personal computing device associated with the user for display to the user. Taking the action may include generating a coaching recommendation for the user. Other embodiments of this example may also or instead include a method and/or a computer program product.

In an example, a method disclosed herein may include: receiving motion data from one or more motion sensors of a wearable fitness monitor worn by a user during a strength training activity; calculating an individualized musculoskeletal strain for the user during the strength training activity by adjusting an intensity associated with the motion data according to an exercise history for the user associated with the strength training activity, an effective load during the strength training activity, and a maximum volume for the user associated with the strength training activity; and taking an action based on the individualized musculoskeletal strain. Other embodiments of this aspect may also or instead include a computer program product comprising computer executable code embodied in a non-transitory computer readable medium that, when executing on one or more computing devices, causes the one or more computing devices to perform one or more of the aforementioned steps. Other embodiments of this example may also or instead include a system having a wearable fitness monitor including one or more motion sensors and one or more processors configured to calculate a user-specific musculoskeletal strain score for a user of the wearable fitness monitor by performing one or more of the aforementioned steps.

In an example, a method disclosed herein may include: receiving motion data from one or more motion sensors of a wearable fitness monitor worn by a user during a strength training activity; calculating a raw intensity score for a number of repetitions of the strength training activity based on the motion data; calculating an effective load for the strength training activity based on one or more load parameters including at least a body weight of the user and an added weight for the strength training activity; calculating an individualized musculoskeletal strain score based on a combination of the raw intensity score calculated from the motion data and the effective load calculated based on the one or more load parameters; and presenting information to the user based on the individualized musculoskeletal strain score. Other embodiments of this example may also or instead include a system having a wearable fitness monitor including one or more motion sensors and one or more processors configured to calculate a user-specific musculoskeletal strain score for a user of the wearable fitness monitor by performing one or more of the aforementioned steps.

BRIEF DESCRIPTION OF THE DRAWINGS

The foregoing and other objects, features, and advantages of the devices, systems, and methods described herein will be apparent from the following description of particular embodiments thereof, as illustrated in the accompanying drawings. The drawings are not necessarily to scale, emphasis instead being placed upon illustrating the principles of the devices, systems, and methods described herein. In the drawings, like reference numerals generally identify corresponding elements.

Fig. 1 shows a physiological monitoring device.
Fig. 2 illustrates a physiological monitoring system.
Fig. 3 shows a sensing system.
Fig. 4 shows a method for generating and using musculoskeletal (MSK) load data.
Fig. 5 shows a load-repetition profile for scaling intensity of exercise repetitions.
Fig. 6 shows a graph of velocity over time measured during a bench press exercise.
Fig. 7 shows three-axis acceleration data for one repetition of the bench press exercise.
Fig. 8 shows an integral of normalized acceleration magnitudes for the data from Fig. 7.
Fig. 9 shows a system for monitoring MSK strain.
Fig. 10 shows a system for monitoring MSK strain.
Fig. 11 shows a system for monitoring MSK strain.
Fig. 12 shows a user interface for a strength training system with MSK strain scoring.
Fig. 13 shows a user interface for a strength training system with MSK strain scoring.

DESCRIPTION

The embodiments will now be described more fully hereinafter with reference to the accompanying figures, in which preferred embodiments are shown. The foregoing may, however, be embodied in many different forms and should not be construed as limited to the illustrated embodiments set forth herein. Rather, these illustrated embodiments are provided so that this disclosure will convey the scope to those skilled in the art.

References to items in the singular should be understood to include items in the plural, and vice versa, unless explicitly stated otherwise or clear from the text. Grammatical conjunctions are intended to express any and all disjunctive and conjunctive combinations of conjoined clauses, sentences, words, and the like, unless otherwise stated or clear from the context. Thus, the term "or" should generally be understood to mean "and/or" and so forth.

Recitation of ranges of values herein are not intended to be limiting, referring instead individually to any and all values falling within the range, unless otherwise indicated, and each separate value within such a range is incorporated into the specification as if it were individually recited herein. The words "about," "approximately," or the like, when accompanying a numerical value, are to be construed as indicating a deviation as would be appreciated by one of ordinary skill in the art to operate satisfactorily for an intended or stated purpose. Similarly, words of approximation such as "approximately" or "substantially" when used in reference to physical characteristics, should be understood to contemplate a range of deviations that would be appreciated by one of ordinary skill in the art to operate satisfactorily for a corresponding use, function, purpose, or the like. Ranges of values and/or numeric values are provided herein as examples only, and do not constitute a limitation on the scope of the described embodiments. Where ranges of values are provided, they are also intended to include each value within the range as if set forth individually, unless expressly stated to the contrary. The use of any and all examples, or exemplary language ("e.g.," "such as," or the like) provided herein, is intended merely to better describe the embodiments and does not pose a limitation on the scope of the disclosed embodiments. No language in the specification should be construed as indicating any unclaimed element as essential to the practice of the embodiments.

In the following description, it is understood that terms such as "first," "second," "top," "bottom," "up," "down," "above," "below," and the like, are words of convenience and are not to be construed as limiting terms unless specifically stated to the contrary.

The term "user" as used herein, refers to any type of animal, human or non-human, whose physiological information may be monitored using an exemplary wearable physiological monitoring system.

The term "continuous," as used herein in connection with heart rate data, refers to the acquisition of heart rate data at a sufficient frequency to enable detection of individual heartbeats, and also refers to the collection of heart rate data over extended periods such as an hour, a day or more (including acquisition throughout the day and night). More generally with respect to physiological signals that might be monitored by a wearable device, "continuous" or "continuously" will be understood to mean continuously at a rate and duration suitable for the intended time-based processing, and physically at an inter-periodic rate (e.g., multiple times per heartbeat, respiration, and so forth) sufficient for resolving the desired physiological characteristics such as heart rate, heart rate variability, heart rate peak detection, pulse shape, and so forth. At the same time, continuous monitoring is not intended to exclude ordinary data acquisition interruptions such as temporary displacement of monitoring hardware due to sudden movements, changes in external lighting, loss of electrical power, physical manipulation and/or adjustment by a wearer, physical displacement of monitoring hardware due to external forces, and so forth. It will also be noted that heart rate data or a monitored heart rate, in this context, may more generally refer to raw sensor data such as optical intensity signals, or processed data therefrom such as heart rate data, signal peak data, heart rate variability data, or any other physiological or digital signal suitable for recovering heart rate information as contemplated herein. Furthermore, such heart rate data may generally be captured over some historical period that can be subsequently correlated to various other data or metrics related to, e.g., sleep states, recognized exercise activities, resting heart rate, maximum heart rate, and so forth.

The term "computer-readable medium," as used herein, refers to a non-transitory storage media such as storage hardware, storage devices, computer memory that may be accessed by a controller, a microcontroller, a microprocessor, a computational system, or the like, or any other module or component or module of a computational system to encode thereon computer-executable instructions, software programs, and/or other data. The "computer-readable medium" may be accessed by a computational system or a module of a computational system to retrieve and/or execute the computer-executable instructions or software programs encoded on the medium. The non-transitory computer-readable media may include, but are not limited to, one or more types of hardware memory, non-transitory tangible media (for example, one or more magnetic storage disks, one or more optical disks, one or more USB flash drives), virtual or physical computer system memory, physical memory hardware such as random access memory (such as, DRAM, SRAM, EDO RAM), and so forth. Although not depicted, any of the devices or components described herein may include a computer-readable medium or other memory for storing program instructions, data, and the like.

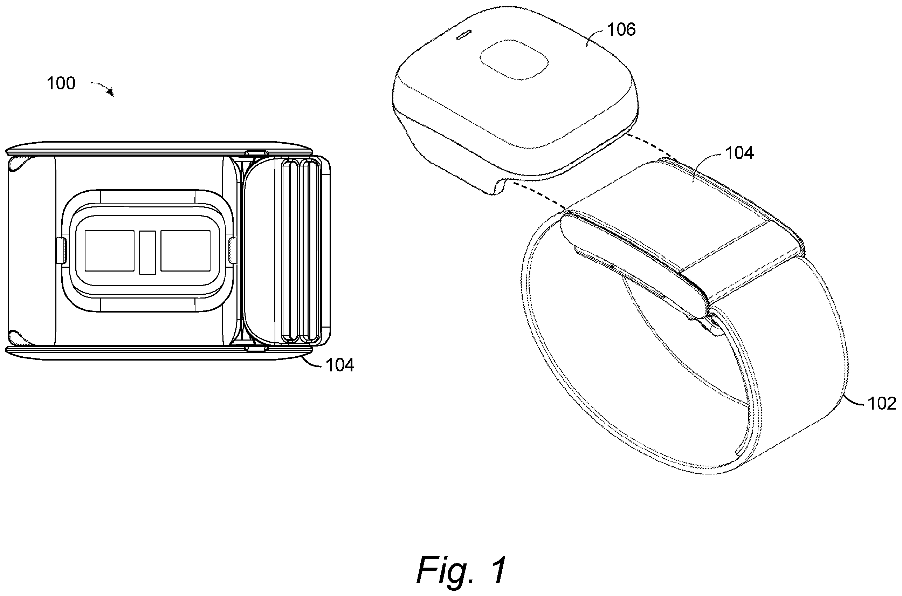

Fig. 1 shows a physiological monitoring system. The system 100 may include a wearable monitor 104 that is configured for physiological monitoring. The system 100 may also include a removable and replaceable battery 106 for recharging the wearable monitor 104. The wearable monitor 104 may include a strap 102 or other retaining system(s) for securing the wearable monitor 104 in a position on a wearer's body for the acquisition of physiological data as described herein. For example, the strap 102 may include a slim elastic band formed of any suitable elastic material such as a rubber or a woven polymer fiber such as a woven polyester, polypropylene, nylon, spandex, and so forth. The strap 102 may be adjustable to accommodate different wrist sizes, and may include any latches, hasps, or the like to secure the wearable monitor 104 in an intended position for monitoring a physiological signal. While a wrist-worn device is depicted, it will be understood that the wearable monitor 104 may be configured for positioning in any suitable location on a user's body, based on the sensing modality and the nature of the signal to be acquired. For example, the wearable monitor 104 may be configured for use on a wrist, an ankle, a bicep, a chest, or any other suitable location(s), and the strap 102 may be, or may include, a waistband or other elastic band or the like within an article of clothing or accessory. The wearable monitor 104 may also or instead be structurally configured for placement on or within a garment, e.g., permanently or in a removable and replaceable manner. To that end, the wearable monitor 104 may be shaped and sized for placement within a pocket, slot, and/or other housing that is coupled to or embedded within a garment. In such configurations, the pocket or other retaining arrangement on the garment may include sensing windows or the like so that the wearable monitor 104 can operate while placed for use in the garment. United States Pat. No. 11,185,292 describes non-limiting example embodiments of suitable wearable monitors 104.

The system 100 may include any hardware components, subsystems, and the like to support various functions of the wearable monitor 104 such as data collection, processing, display, and communications with external resources. For example, the system 100 may include hardware for a heart rate monitor using, e.g., photoplethysmography, electrocardiography, or any other technique(s). The system 100 may be configured such that, when the wearable monitor 104 is placed for use about a wrist (or at some other body location), the system 100 initiates acquisition of physiological data from the wearer. In some embodiments, the pulse or heart rate may be acquired optically based on a light source (such as light emitting diodes (LEDs)) and optical detectors in the wearable monitor 104. The LEDs may be positioned to direct illumination toward the user's skin, and optical detectors such as photodiodes may be used to capture illumination intensity measurements indicative of illumination from the LEDs that is reflected and/or transmitted by the wearer's skin.

The system 100 may be configured to record other physiological and/or biomechanical parameters including, but not limited to, skin temperature (using a thermometer), galvanic skin response (using a galvanic skin response sensor), motion (using one or more multi-axes accelerometers and/or gyroscope), blood pressure, and the like, as well environmental or contextual parameters such as ambient light, ambient temperature, humidity, time of day, and so forth. For example, the wearable monitor 104 may include sensors such as accelerometers and/or gyroscopes for motion detection, sensors for environmental temperature sensing, sensors to measure electrodermal activity (EDA), sensors to measure galvanic skin response (GSR) sensing, and so forth. The system 100 may also or instead include other systems or subsystems supporting addition functions of the wearable monitor 104. For example, the system 100 may include communications systems to support, e.g., near field communications, proximity sensing, Bluetooth communications, Wi-Fi communications, cellular communications, satellite communications, and so forth. The wearable monitor 104 may also or instead include components such as a GeoPositioning System (GPS), a display and/or user interface, a clock and/or timer, and so forth.

The wearable monitor 104 may include one or more sources of battery power, such as a first battery within the wearable monitor 104 and a second battery 106 that is removable from and replaceable to the wearable monitor 104 in order to recharge the battery in the wearable monitor 104. Also or instead, the system 100 may include a plurality of wearable monitors 104 (and/or other physiological monitors) that can share battery power or provide power to one another. The system 100 may perform numerous functions related to continuous monitoring, such as automatically detecting when the user is asleep, awake, exercising, and so forth, and such detections may be performed locally at the wearable monitor 104 or at a remote service coupled in a communicating relationship with the wearable monitor 104 and receiving data therefrom. In general, the system 100 may support continuous, independent monitoring of a physiological signal such as a heart rate, and the underlying acquired data may be stored on the wearable monitor 104 for an extended period until it can be uploaded to a remote processing resource for more computationally complex analysis.

In one aspect, the wearable monitor may be a wrist-worn photoplethysmography device.

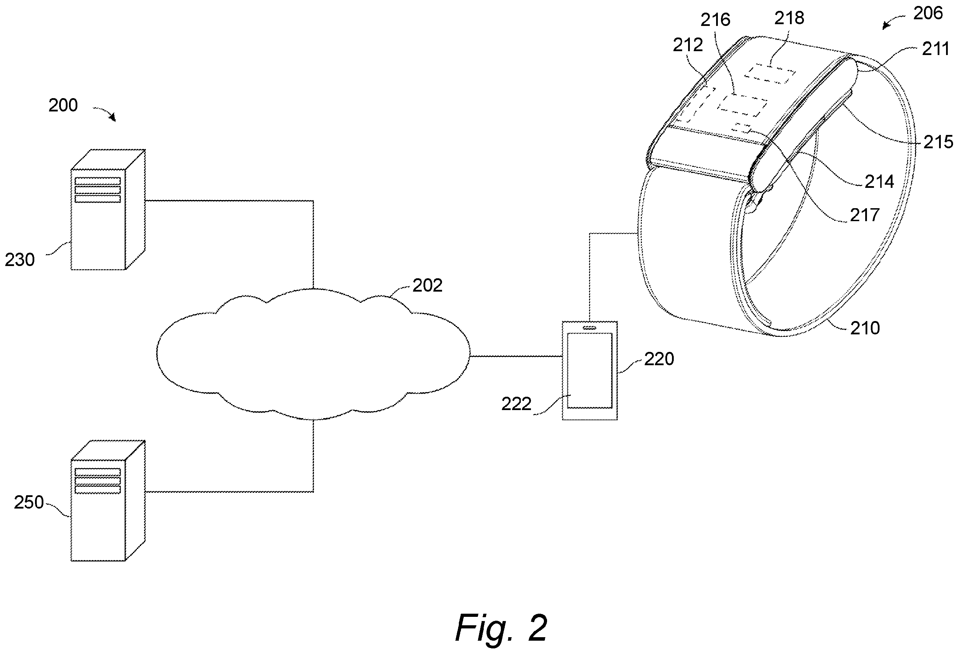

Fig. 2 illustrates a physiological monitoring system. More specifically, Fig. 2 illustrates a physiological monitoring system 200 that may be used with any of the methods or devices described herein. In general, the system 200 may include a physiological monitor 206, a user device 220, a remote server 230 with a remote data processing resource (such as any of the processors or processing resources described herein), and one or more other resources 250, all of which may be interconnected through a data network 202.

The data network 202 may be any of the data networks described herein. For example, the data network 202 may be any network(s) or internetwork(s) suitable for communicating data and information among participants in the system 200. This may include public networks such as the Internet, private networks, telecommunications networks such as the Public Switched Telephone Network or cellular networks using third generation (e.g., 3G or IMT-200), fourth generation (e.g., LTE (E-UTRA) or WiMAX-Advanced (IEEE 802.16m)), fifth generation (e.g., 5G), and/or other technologies, as well as any of a variety of corporate area or local area networks and other switches, routers, hubs, gateways, and the like that might be used to carry data among participants in the system 200. This may also include local or short-range communications infrastructure suitable, e.g., for coupling the physiological monitor 206 to the user device 220, or otherwise supporting communicating with local resources. By way of non-limiting examples, short range communications may include Wi-Fi communications, Bluetooth communications, infrared communications, near field communications, communications with RFID tags or readers, and so forth.

The physiological monitor 206 may, in general, be any physiological monitoring device or system, such as any of the wearable monitors or other monitoring devices or systems described herein. In one aspect, the physiological monitor 206 may be a wearable physiological monitor shaped and sized to be worn on a wrist or other body location. The physiological monitor 206 may include a wearable housing 211, a network interface 212, one or more sensors 214, one or more light sources 215, a processor 216, a haptic device 217 or other user input/output hardware, a memory 218, and a strap 210 for retaining the physiological monitor 206 in a desired location on a user. In one aspect, the physiological monitor 206 may be configured to acquire heart rate data and/or other physiological data from a wearer in an intermittent or substantially continuous manner. In another aspect, the physiological monitor 206 may be configured to support extended, continuous acquisition of physiological data, e.g., for several days, a week, or more.

The network interface 212 of the physiological monitor 206 may be configured to couple the physiological monitor 206 to one or more other components of the system 200 in a communicating relationship, either directly, e.g., through a cellular data connection or the like, or indirectly through a short range wireless communications channel coupling the physiological monitor 206 locally to a wireless access point, router, computer, laptop, tablet, cellular phone, or other device that can locally process data, and/or relay data from the physiological monitor 206 to the remote server 230 or other resource(s) 250 as necessary or helpful for acquiring and processing data from the physiological monitor 206.

The one or more sensors 214 may include any of the sensors described herein, or any other sensors or subsystems suitable for physiological monitoring or supporting functions. By way of example and not limitation, the one or more sensors 214 may include one or more of a light source, an optical sensor, an accelerometer, a gyroscope, a temperature sensor, a galvanic skin response sensor, a capacitive sensor, a resistive sensor, an environmental sensor (e.g., for measuring ambient temperature, humidity, lighting, and the like), a geolocation sensor, a Global Positioning System, a proximity sensor, an RFID tag reader, and RFID tag, a temporal sensor, an electrodermal activity sensor, and the like. The one or more sensors 214 may be disposed in the wearable housing 211, or otherwise positioned and configured for physiological monitoring or other functions described herein. In one aspect, the one or more sensors 214 include a light detector configured to provide light intensity data to the processor 216 (or to the remote server 230) for calculating a heart rate and a heart rate variability. The one or more sensors 214 may also or instead include an accelerometer, gyroscope, and the like configured to provide motion data to the processor 216, e.g., for detecting activities such as a sleep state, a resting state, a waking event, exercise, and/or other user activity. In an implementation, the one or more sensors 214 may include a sensor to measure a galvanic skin response of the user. The one or more sensors 214 may also or instead include electrodes or the like for capturing electronic signals, e.g., to obtain an electrocardiogram and/or other electrically-derived physiological measurements.

The processor 216 and memory 218 may be any of the processors and memories described herein. In one aspect, the memory 218 may store physiological data obtained by monitoring a user with the one or more sensors 214, and or any other sensor data, program data, or other data useful for operation of the physiological monitor 206 or other components of the system 200. It will be understood that, while only the memory 218 on the physiological monitor is illustrated, any other device(s) or components of the system 200 may also or instead include a memory to store program instructions, raw data, processed data, user inputs, and so forth. In one aspect, the processor 216 of the physiological monitor 206 may be configured to obtain heart rate data from the user, such as heart rate data including or based on the raw data from the sensors 214. The processor 216 may also or instead be configured to determine, or assist in a determination of, a condition of the user related to, e.g., health, fitness, strain, recovery sleep, or any of the other conditions described herein.

The one or more light sources 215 may be coupled to the wearable housing 211 and controlled by the processor 216. At least one of the light sources 215 may be directed toward the skin of a user adjacent to the wearable housing 211. Light from the light source 215, or more generally, light at one or more wavelengths of the light source 215, may be detected by one or more of the sensors 214, and processed by the processor 216 as described herein.

The system 200 may further include a remote data processing resource executing on a remote server 230. The remote data processing resource may include any of the processors and related hardware described herein, and may be configured to receive data transmitted from the memory 218 of the physiological monitor 206, and to process the data to detect or infer physiological signals of interest such as heart rate, heart rate variability, respiratory rate, pulse oxygen, blood pressure, and so forth. The remote server 230 may also or instead evaluate a condition of the user such as a recovery state, sleep state, exercise activity, exercise type, sleep quality, daily activity strain, and any other health or fitness conditions that might be detected based on such data.

The system 200 may include one or more user devices 220, which may work together with the physiological

EP 4 358 838 B1

monitor 206, e.g., to provide a display, or more generally, user input/output, for user data and analysis, and/or to provide a communications bridge from the network interface 212 of the physiological monitor 206 to the data network 202 and the remote server 230. For example, physiological monitor 206 may communicate locally with a user device 220, such as a smartphone of a user, via short-range communications, e.g., Bluetooth, or the like, for the exchange of data between the physiological monitor 206 and the user device 220, and the user device 220 may in turn communicate with the remote server 230 via the data network 202 in order to forward data from the physiological monitor 206 and to receive analysis and results from the remote server 230 for presentation to the user. In one aspect, the user device(s) 220 may support physiological monitoring by processing or pre-processing data from the physiological monitor 206 to support extraction of heart rate or heart rate variability data from raw data obtained by the physiological monitor 206. In another aspect, computationally intensive processing may advantageously be performed at the remote server 230, which may have greater memory capabilities and processing power than the physiological monitor 206 and/or the user device 220.

The user device 220 may include any suitable computing device(s) including, without limitation, a smartphone, a desktop computer, a laptop computer, a network computer, a tablet, a mobile device, a portable digital assistant, a cellular phone, a portable media or entertainment device, or any other computing devices described herein. The user device 220 may provide a user interface 222 for access to data and analysis by a user, and/or to support user control of operation of the physiological monitor 206. The user interface 222 may be maintained by one or more applications executing locally on the user device 220, or the user interface 222 may be remotely served and presented on the user device 220, e.g., from the remote server 230 or the one or more other resources 250.

In general, the remote server 230 may include data storage, a network interface, and/or other processing circuitry. The remote server 230 may process data from the physiological monitor 206 and perform physiological and/or health monitoring/analyses or any of the other analyses described herein, (e.g., analyzing sleep, determining strain, assessing recovery, and so on), and may host a user interface for remote access to this data, e.g., from the user device 220. The remote server 230 may include a web server or other programmatic front end that facilitates web-based access by the user devices 220 or the physiological monitor 206 to the capabilities of the remote server 230 or other components of the system 200.

The system 200 may include other resources 250, such as any resources that can be usefully employed in the devices, systems, and methods as described herein. For example, these other resources 250 may include other data networks, databases, processing resources, cloud data storage, data mining tools, computational tools, data monitoring tools, algorithms, and so forth. In another aspect, the other resources 250 may include one or more administrative or programmatic interfaces for human actors such as programmers, researchers, annotators, editors, analysts, coaches, and so forth, to interact with any of the foregoing. The other resources 250 may also or instead include any other software or hardware resources that may be usefully employed in the networked applications as contemplated herein. For example, the other resources 250 may include payment processing servers or platforms used to authorize payment for access, content, or option/feature purchases. In another aspect, the other resources 250 may include certificate servers or other security resources for third-party verification of identity, encryption or decryption of data, and so forth. In another aspect, the other resources 250 may include a desktop computer or the like co-located (e.g., on the same local area network with, or directly coupled to through a serial or USB cable) with a user device 220, wearable strap 210, or remote server 230. In this case, the other resources 250 may provide supplemental functions for components of the system 200 such as firmware upgrades, user interfaces, and storage and/or pre-processing of data from the physiological monitor 206 before transmission to the remote server 230.

The other resources 250 may also or instead include one or more web servers that provide web-based access to and from any of the other participants in the system 200. While depicted as a separate network entity, it will be readily appreciated that the other resources 250 (e.g., a web server) may also or instead be logically and/or physically associated with one of the other devices described herein, and may for example, include or provide a user interface 222 for web access to the remote server 230 or a database or other resource(s) to facilitate user interaction through the data network 202, e.g., from the physiological monitor 206 or the user device 220.

In another aspect, the other resources 250 may include fitness equipment or other fitness infrastructure. For example, a strength training machine may automatically record repetitions and/or added weight during repetitions, which may be wirelessly accessible by the physiological monitor 206 or some other user device 220. More generally, a gym may be configured to track user movement from machine to machine, and report activity from each machine in order to track various strength training activities in a workout. The other resources 250 may also or instead include other monitoring equipment or infrastructure. For example, the system 200 may include one or more cameras to track motion of free weights and/or the body position of the user during repetitions of a strength training activity or the like. Similarly, a user may wear, or have embedded in clothing, tracking fiducials such as visually distinguishable objects for image-based tracking, or radio beacons or the like for other tracking. In another aspect, weights may themselves be instrumented, e.g., with sensors to record and communicated detected motion, and/or beacons or the like to self-identify type, weight, and so forth, in order to facilitate automated detection and tracking of exercise activity with other connected devices.

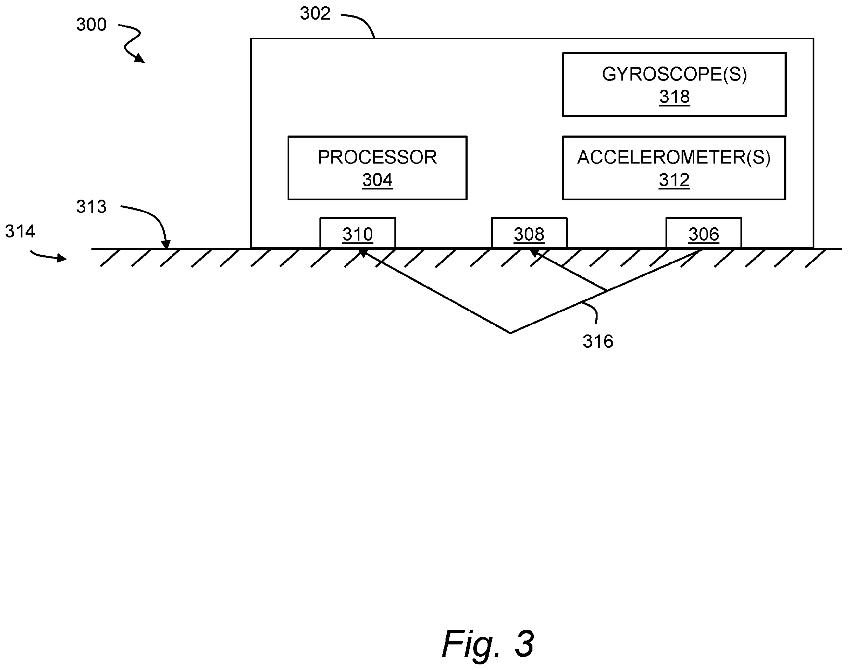

Fig. 3 shows a sensing system. In general, the system 300 may include a physiological monitor 302 with a

8

processor 304, a light source 306, a first photodetector 308, a second photodetector 310, one or more accelerometers 312, one or more gyroscopes 318, and any other hardware or other components and systems suitable for physiological monitoring as described herein. The physiological monitor 302 may be positioned for use against a surface 313 of the skin 314 of a user where the light source 306 and sensors 308, 310 can contact the skin 314 for acquisition of physiological data. Although not depicted, it will be understood that the physiological monitor 302 may generally be retained in position using any of the straps, garments, or the like described herein.

The processor 304 may be any microprocessor, microcontroller, application specific integrated circuit, or other processing circuitry or combination of the foregoing suitable for controlling operation of the physiological monitor and acquiring physiological data.

The light source 306 may include one or more light emitting diodes or other sources of illumination, and may be positioned within the physiological monitor 302 such that, when the physiological monitor 302 is placed for use on the skin 314, the light source 306 directs illumination toward the skin 314 and the illumination is reflected back toward the sensors 308, 310 as indicated by arrows 316, where the intensity can be measured. In one aspect, the light source 306 may include light emitting diodes that emit light in the infrared or near infrared wavelength ranges, which provides good light transmission through human skin, facilitating low-power transmission of measurable illumination to the sensors 308, 310, although other illumination sources and wavelengths may also or instead be used.

The sensors 308, 310 may be oriented to contact the skin 314 when the physiological monitor 302 is placed for use on this skin 314, and positioned so that the sensors 308, 310 can capture illumination reflected and/or transmitted by the skin from the light source 306. In general, the sensors 308, 310 may include photodiodes, photodetectors, or any other sensor(s) responsive to illumination from the light source 306. This may include broadband optical sensors, narrowband optical sensors, filtered sensors, or the like. In general, a first sensor 308 may be positioned closer to the light source 306 than a second sensor 310 to facilitate detection of differential intensity in the measured wavelength(s). For example, the first sensor 308 may be positioned 1-4 millimeters from the light source 306 and the second sensor 310 may be positioned 2-8 millimeters from the light source, or about twice as far as the first sensor 310 from the light source 306.

Other spacings may also or instead be used depending on, e.g., the intensity of the light source 306, the sensitivity of the sensors 308, 310, the contact force of the physiological monitor 302 on the skin 314, the degree of incursion of ambient light, the physiological measurements/properties of interest, and so forth. In one aspect, the sensors 308, 310 may be linearly arranged in a straight line away from the light source 306. While this provides consistency in comparative measurements, it is not strictly required, and the sensors 308 may be displaced in any directions away from the light source 306 provided they both contact the skin 314 in a manner that permits capture of light through the skin 314 from the light source 306. In another aspect, the physiological monitor 302 may include one or more other light sources and/or light sensors, which may be arranged to improve accuracy and/or provide redundancy for the contact detection, or to support other measurements such as oxygenation or skin thickness. This may include light sources/sensors using different ranges of wavelengths, different patterns of illumination, and so forth. In another aspect, the two sensors 308, 310 may be positioned at different distances from a perimeter of the physiological monitor 302 so that the sensors 308, 310 can acquire differential intensity values for ambient light incident on the skin and transmitted through the skin to the sensors 308, 310.

In operation, the processor 304 may acquire raw intensity data from the sensors 308, 310, and perform local calculations such as pre-processing raw data for heart rate measurements, or evaluating whether the physiological monitor 302 is properly placed for use on the skin 314.

The accelerometer 312 may include, e.g., one or more single axis or multi-axis accelerometers, which may usefully measure motion of the physiological monitor 302 to support calculations such as automated activity detection, device on/off evaluation, and degree of musculoskeletal activation, e.g., as described herein. Other motion and orientation sensing hardware-such as one or more gyroscopes 318, inertial motion sensors, and/or other micro-electromechanical system (MEMS) sensors-may also or instead be used for these purposes. More generally, the physiological monitor 302 may include any additional components, subsystems, and the like suitable for supporting various modes of physiological monitoring and contextual data acquisition as described herein.

Methods for calculating a strain score based on heart rate are described by way of non-limiting examples, in U.S. Pat. No. 11,185,292. In one aspect, there is disclosed herein additional methods and systems for estimating musculoskeletal load based on patterns of motion in a monitoring device, and using this load to provide improved strain analysis, coaching recommendations, and the like. Musculoskeletal (MSK) strain captures a type of exertion that may be missed or underestimated when estimating strain based on heart rate alone. Motion data such as accelerometer data or gyroscope data from a wearable device may advantageously be used to fill this void and estimate muscular strain based on various motion parameters during strength training activity. While it is possible for a user to record weight, repetitions, and subjective strain, a direct objective measurement of MSK strain advantageously relieves a user of manual data entry, mitigates underreporting of high strain events, removes subjective variability from strain calculations, and so forth. In one aspect, the disclosed techniques include objectively quantifying the effort of a strength training exercise-as well as other activities-and reporting corresponding strain metrics that can be used to understand the physiological impact of strength training activities. In another aspect, the disclosed techniques may be used to create coaching metrics, refinements to cardiac-based strain estimates, and so forth. In another aspect, the disclosed techniques may be used to monitor whether exercise techniques or metrics-e.g., weights currently being used in a strength training exercise-are appropriate given the intended training stimulus and/or a user's goals (e.g., toning, gains, weight loss, etc.).

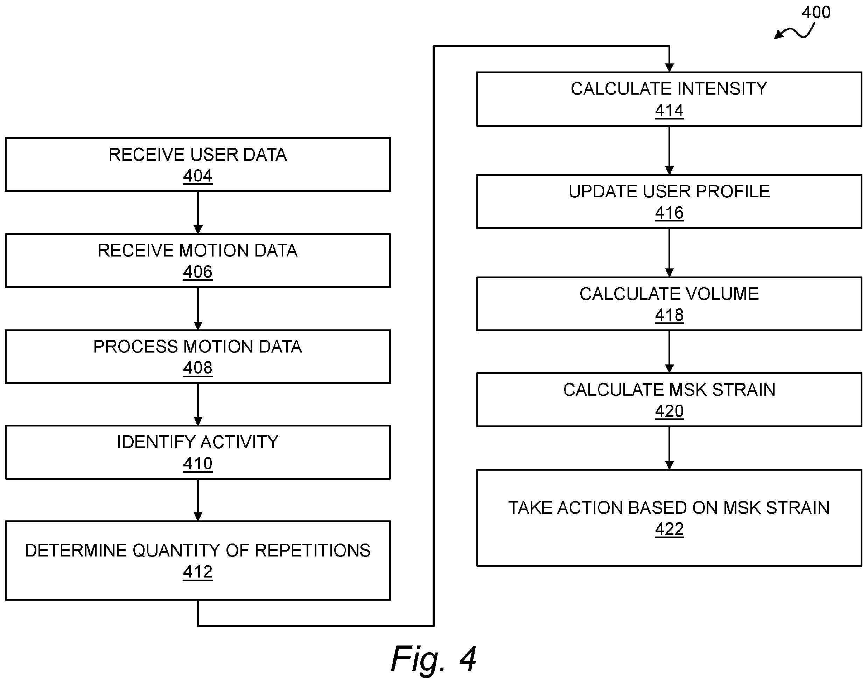

Fig. 4 shows a method for calculating a musculoskeletal (MSK) strain score. The method 400 may be deployed, for example, on any of the devices and systems described herein, and may be deployed with computer code for performing some or all of the following steps. An MSK strain may be evaluated as two components-volume and intensity. As described herein an objective measure of MSK strain may be obtained by developing metrics for measuring each of these components during strength training activity and combining them into a single MSK strain score that can be reported to a user, and/or used for coaching recommendations or the like.

In the context of strength training, "volume" generally refers to the total amount of work done in a given workout or over a specific period of time. There are different ways to calculate volume, but one common method is to multiply the number of sets for a particular exercise by the number of repetitions in each set, and then by the weight lifted in each repetition. For example, for 3 sets of 10 reps with 100 pounds, the volume would be 3,000 pounds (3 sets x 10 reps x 100 pounds). Another way to consider volume is simply the total number of sets or reps performed for a certain muscle group or exercise in a workout. For example, for 5 sets of 5 reps on the bench press, the volume would be 25 reps (5 sets x 5 reps). It is important to manage volume in strength training because it has a major impact on recovery and progress. Too much volume can lead to overtraining and increased risk of injury, while too little may not provide enough stimulus for growth and improvement.

As described below, volume-the total work done in a strength training activity-may be objectively quantified for a particular user by, e.g., determining the effective load of an exercise for a user, and comparing this to a maximum volume for the user. The effective load may be based on other objective parameters such as physical weights lifted by a user (e.g., pounds on a barbell, pounds of a hand weight, pounds on a strength machine, etc.) or the body weight of the user (which may affect exercises such as pushups, squats, pullups, and so forth, where the weight of the user is providing some or all of the load), or some combination of these (e.g., where a user is performing pullups with ten added pounds of weight). The maximum volume may be estimated for the user based on, e.g., a statistical estimate of the number of repetitions at a particular weight/volume at which an injury becomes more likely.

The notion of intensity presents different computational challenges. In strength training, "intensity" generally refers to the amount of effort or load for an exercise relative to a maximum capability, or how difficult a particular load is for a particular individual. This is often defined as a percentage of a one-repetition maximum (1RM), which is the maximum amount of weight that can be lifted for one repetition of a particular exercise. For example, if a user has a 1RM for a bench press of 200 pounds and is lifting 150 pounds, the user is training at an intensity of 75% of their 1RM (150 divided by 200). Another way to measure intensity, especially in methods like high-intensity interval training (HIIT), is through perceived exertion or how hard the exercise feels. This can be somewhat subjective, but tools like the Borg Rating of Perceived Exertion (RPE) scale can help to quantify it.

In order to capture intensity-e.g., muscular capacity measured relative to a maximum-physical movements during an exercise may be tracked by a wearable monitor and used to objectively calculate a motion-based intensity for a particular user and exercise type. Because different users have different capacity, this motion-based metric may be scaled according to a user exercise history to determine how much effort, relative to a maximum, the user has exerted during an exercise. It may also be useful, in some cases, to synthesize an intensity based on other data. For example, some exercises may not involve motion that can be detected by a wearable monitor, such as training on a leg curl machine or leg extension machine while using a wrist-worn monitor. In these cases, a user may report weight and repetitions, and intensity may be estimated based on user history. In another aspect, some exercises do not involve motion at all. For example, isometric exercises such as planks or wall sits involve remaining stationary. For these exercises, a "repetition" may be derived based on an amount of time that the exercise is performed. For example, a plank may be represented with one repetition every six seconds. In some cases, both of these techniques may be used, e.g., where a user is performing an isometric exercise that loads a muscle group whose movement (e.g., shaking due to strain) cannot be detected at a location of a wearable monitor. In other cases, muscular shaking due to isometric load may be detectable by a wearable monitor and used to evaluate strain, even if no motion is intended by the user.

An example method is described below for calculating a musculoskeletal strain based on objective measures of intensity and volume.

As shown in step 404, the method may include receiving user data. In one aspect, this may include receiving input from a user such as body weight, added weight, and other parameters for evaluating a strength training activity. This may also include manually entered information such as weight, repetitions, and type for one or more strength training activities. For manual data entry, this may be entered by the user on a user device before, during, and/or after a workout, or may be entered on a wearable monitor with a suitable user interface for corresponding data entry. For some activities, it may be difficult, impossible, or inconvenient for the wearable monitor to track exercise. For example, a user performing an isometric exercise that does not load a muscle near the wearable monitor may be difficult or impossible to measure automatically with the wearable monitor. In these cases, a user may manually enter some or all of the related data in order to support MSK strain analysis for a workout.

In another aspect, some or all of the user data that describes a particular workout or strength training activity may be automatically derived from, e.g., motion data acquired by a wearable monitor or smart gym equipment. For example, a wearable monitor may detect an activity type based on characteristics of motion data acquired by the wearable monitor during exercise. The wearable monitor may also or instead detect individual repetitions in a set. In another aspect, the user may provide a per repetition or per set input to demarcate activities, which may be used by a system such as any described herein to more readily identify sets, as well as repetitions in a set.

In another aspect, data may be obtained from other equipment. For example, a strength training machine may count repetitions and wirelessly or otherwise report these repetitions to the wearable monitor or some other user device. The strength training machine may also or instead report an amount of weight for a particular set of repetitions, which may be retrieved by the wearable monitor or other device and used to support MSK strain calculations as described herein. In another aspect, a camera may be used to track user motion and/or equipment, and may be used to derive strength training data from camera images. For example, image processing may be applied to identify an activity, count repetitions, evaluate form, identify added weights, and so forth. In another aspect, weights may be instrumented or tagged to support acquisition of motion data, weight data, and the like directly from the weights. Combinations may also be used. For example, a camera may be used to count repetitions, while weights may be tagged to permit automated identification for determination of load.

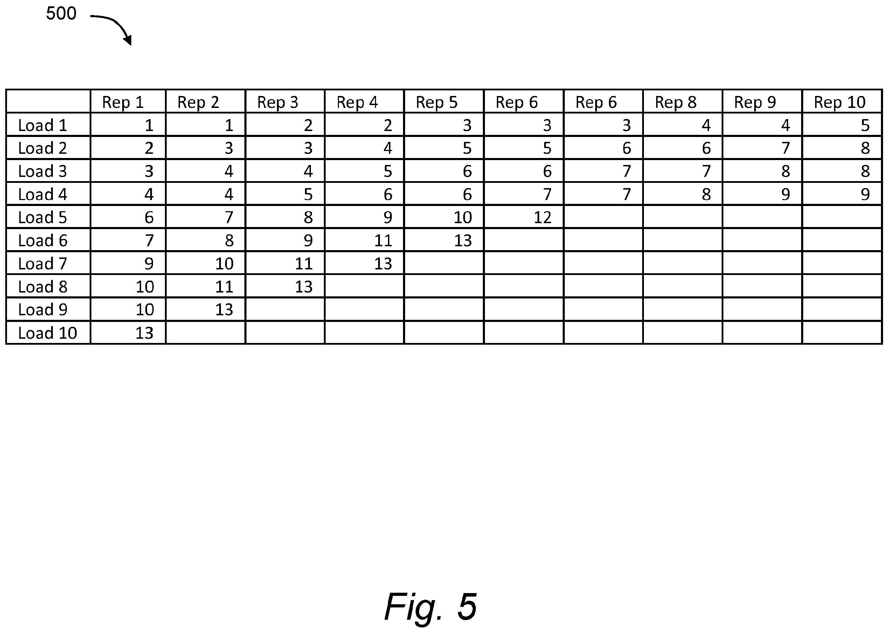

In one aspect, user data includes historical exercise data for the user. This may be retrieved, e.g., from a remote server or other resource, and may be used to support MSK score calculations. For example, this may include retrieving a load-repetition profile for the user based on a history of strength training activity by the user, such as the load-repetition profile illustrated in Fig. 5. The load-repetition profile may generally indicate a capacity for repetitions by the user at one or more loads during the strength training activity and may be used to scale a raw intensity for the user based on motion data from the wearable device. While it is known in the art to use velocity of repetitions to detect the approach of a maximum, a load-repetition profile as contemplated herein may advantageously use acceleration data to facilitate estimates of intensity using data from sensors in a wearable device. If no load-repetition profile is available for the user, the method 400 may include creating the load-repetition profile. The method 400 may also include updating the profile as appropriate. For example, the method may include adding a row to the load-repetition profile when the user performs a strength training activity at a new load that is not included in the one or more loads in the load-repetition profile. The method 400 may also or instead include adding a column when the user does a new number of repetitions, or updating the load-repetition profile when the user exceeds a number of repetitions or a load within the load-repetition profile.

In another aspect, a maximum volume for the user may be retrieved, or may be estimated based on other retrieved user data. Calculating the maximum volume for an exercise can be somewhat complex and individualized, as it depends on various factors such as a current fitness level, a specific exercise, a training goal, and how a user responds to different training volumes. As used herein, the maximum volume is intended to provide an indication of an upper threshold for injury-free repetitions of the strength training activity by the user. A variety of techniques may be used to estimate this threshold. For example, in the absence of specific user data, a linear regression can be used with a population of users to derive a formula relating maximum volume to body weight (max. volume = a * body weight + b), which may be used as an estimate before other user data is available. For a significant number of user-specific samples, a maximum volume for safe load may be usefully calculated as the baseline or mean volume per workout plus twice the standard deviation for workout volumes. In another aspect, a progression of techniques may be used based on how many user-specific volume measurements are available. More generally, any useful technique for estimating a threshold or limit for injury-free volume, e.g., a threshold at or below which effort presents an acceptable risk of injury, may be used to calculate a maximum volume for scaling per-workout volumes as described herein.

As shown in step 406, the method may include receiving motion data. This may, for example, include receiving raw motion data from one or more motion sensors of a wearable monitor such as a wearable fitness monitor worn by a user during a strength training activity including a set of one or more repetitions. The raw motion data may include raw motion data from at least one gyroscope and at least one accelerometer of the wearable monitor. More generally, the raw motion data may include any motion data from the motion sensors, including angular rotation data from a plurality of gyroscopes and linear acceleration data from a plurality of accelerometers. In another aspect, receiving motion data may include receiving motion data from numerous body locations, e.g., where a user has dual wrist and/or ankle bands, and/or where the user is wearing a smart garment with suitable motion sensors at various body locations. Motion data may also or instead be received from other sources, such as other external motion sensors, a smart watch or other wearable computing device, an external camera for measuring motion, and so forth.

As shown in step 408, the method 400 may include processing the motion data.

In one aspect, this may include fusing the raw motion data from the one or more motion sensors to mitigate gravitational artifacts, thereby providing motion data including three-axis acceleration data. Data fusion, particularly using sensor fusion techniques, can help mitigate the impact of gravity on accelerometer measurements. Three-axis accelerometers measure both dynamic acceleration (resulting from movement) and static acceleration (the constant force of gravity pulling the device downward). To separate gravity-induced artifacts from motion-related data, other sensors can be used in tandem with the accelerometer, such as a gyroscope or a magnetometer. One popular technique for this is the use of a Kalman filter or an extended Kalman filter, which are recursive algorithms that use a series of measurements observed over time (in this case, the readings from the accelerometer and gyroscope/magnetometer) and produce estimates of unknown variables that tend to be more accurate than those based on any single measurement alone. Another common technique is the use of a complementary filter such as a Mahony or Madgwick filter. These algorithms combine accelerometer and gyroscope data to provide a more stable, accurate, and drift-free measurement of orientation, even in the presence of the constant force of gravity. More generally, by taking readings from multiple sensors and combining them, it is possible to separate a measured acceleration into an acceleration due to motion and an acceleration due to gravity, thus mitigating the effects of gravitational artifacts on the accelerometer's measurements. Any such techniques may be used to process motion data and obtain three-axis acceleration data as described herein.

In general, motion data, as used herein may refer to raw motion data from sensors, fused motion data as described above, filtered motion data, or any other raw or processed data from a wearable monitor representative of motion by the wearer. Thus, in one aspect, motion data may include raw motion data from a three-axis gyroscope and a three-axis accelerometer. In another aspect, motion data may include any such raw data that has been fused to provide three-axis acceleration data for the repetitions with mitigated effects of acceleration due to gravity.

As shown in step 410, the method 400 may include identifying an activity. More specifically, this may include identifying a type of the strength training activity being performed by a user. In one aspect, this may include receiving a user input specifying the type of the strength training activity, such as in the user interface of a user device. In another aspect, this may include automatically identifying the type of the strength training activity based on motion data such as the raw motion data or the three-axis acceleration data or other motion data derived from or based on the raw motion data. Motion data may also or instead be obtained from other sources such as a camera capturing images of the activity, weights or weight training equipment within integrated motion sensors, and so forth. Identifying the activity may, for example, include applying any suitable activity recognition algorithms such as machine learning algorithms, statistical classification schemes, and so forth to the motion data acquired from the wearable monitor or other source. In another aspect, the method 400 may include attempting automatic type detection, and requesting user input in cases where automatic detection cannot reliably (e.g., with a sufficient statistical confidence) identify the type. In one aspect, the method 400 may include continuously tracking motion and attempting to identify known patterns of strength training activity. In another aspect, identifications may only be attempted during known workout times, or in response to an explicit user request.

Where an activity is automatically identified, additional processing may usefully be applied. For example, an activity may be evaluated to determine whether each repetition was completed properly with the full intended range of motion. Certain features of a repetition can also or instead be used to measure repetition-level effort. For example, repetitions that change in velocity, or that include shaking movements, or that stall midway through before being abandoned may suggest greater musculoskeletal effort than would be expected by a smooth repetition performed at a similar pace to the prior repetition. Such variations are often observable in the motion data. A variety of statistical measures such as average signal amplitude, standard deviation, rate of change, and so forth may be used to quantify these variations. The intensity metric may then be compiled based on different statistical quantifications of the motion signals to distinguish among different musculoskeletal exertion levels.

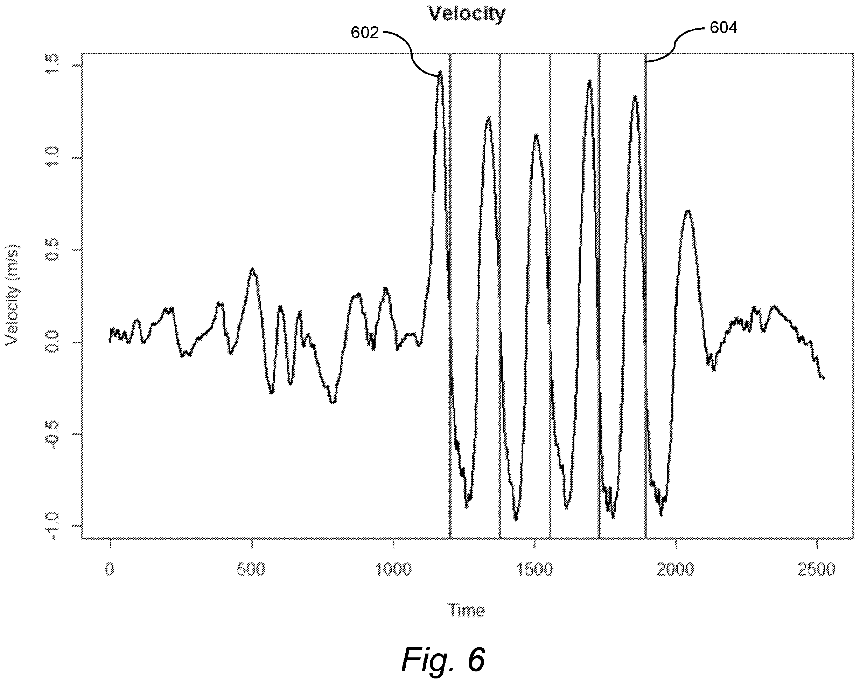

As shown in step 412, the method 400 may include identifying a quantity of the repetitions in a set of the strength training activity. In one aspect, this may include receiving user input specifying a number of repetitions in the set, or otherwise determining the quantity of the repetitions based on user input. In another aspect, this may include determining the quantity of the repetitions in the set based on variations in a magnitude of the three-axis acceleration data, or otherwise determining the quantity based on user input. For example, as shown in Fig. 6, the velocity over time may be derived from the three-axis acceleration data, any may exhibit certain periodic characteristics indicative of repetitions of an exercise repetition. The velocity data may thus be used to support automated detection of repetitions for certain types of exercise. A camera or other tracking device/system may also or instead be used to identify an activity and/or a number of repetitions in an activity. In another aspect, this may include detecting the quantity using other data sources or techniques, such as by receiving a repetition count from exercise equipment, extracting repetition count information from video images of the activity, e.g., as obtained by a smart phone, camera, or other image source, or receiving motion data from any of the other sources described herein.

As shown in step 414, the method may include calculating an intensity for the set.

In one aspect, this may include, for each one of the repetitions, calculating a raw intensity score indicative of musculoskeletal movement based on changes in the three-axis acceleration data. As noted above, intensity generally measures exertion relative to a user's capacity. A raw measure of this intensity may be assessed based on motion data. For example, intensity may be assessed by calculating, for a series of acceleration measurements taken over a repetition, the differential from one instantaneous acceleration measurement to the next (also referred to as "jerk," or the change in acceleration between two measurements), and then dividing this quantity by the acceleration magnitude. The intensity of an exercise repetition may then be calculated by taking the average of this instantaneous intensity for all samples during the concentric phase of the repetition:

$$Intensity_{sample} = \frac{JerkMag_{sample}}{AccMag_{sample}}$$

$$Intensity_{rep} = Mean(Intensity_{sample})$$

It will be understood that other metrics for intensity may be derived based on motion. For example, in one aspect the intensity for a repetition may be calculated as follows:

$$Intensity_{rep} = \frac{Mean(JerkMag_{sample})}{Mean(AccMag_{sample})}$$

This latter approach may, for some exercise types, be less sensitive to small changes in the acceleration or jerk, or to motion changes occurring at concentric phase boundaries. Thus, in one aspect, calculating the raw intensity score for one of the repetitions in a set includes calculating a mean of a number of instantaneous intensity measurements for the one of the repetitions. In one aspect, the one or more of the number of instantaneous intensity measurements is calculated based on a mean of a ratio of discretely measured changes in a current acceleration to the current acceleration. In another aspect, the one or more of the number of instantaneous intensity measurements is calculated based on a ratio of a first mean of a current change in acceleration to a second mean of a current acceleration. More generally, any metric that objectively characterizes intensity based on changes in motion over the course of a repetition of a strength training activity, and/or over a set of such repetitions, may also or instead be used to measure intensity and calculate musculoskeletal strain as contemplated herein. As a significant advantage, intensity measurements based on acceleration permit the capture of spatial range of motion that directly correlates to the work done, as well as any shakiness within the motion indicative of high individual strain. This relatively high speed sporadic motion off the typical path of the exercise will manifest as a quantitatively higher intensity score due to the accumulation of changes in acceleration resulting from the shakiness. For activities where motion cannot be directly measured, a proxy for intensity may be used, such as a duration of a stationary isometric exercise. Even in these cases where there is no gross muscle movement, muscle shakiness may still manifest in a way that can be detected, measured, and used to quantitatively evaluate intensity.

As noted above, calculating intensity may also include adjusting the raw intensity score based on a history of user activity. To individualize the intensity in this manner, the method 400 may include, for each one of the repetitions in a set (or for the entire set), determining a maximum intensity for performing the strength training activity by the user. The maximum intensity may be indicative of a capacity of the user to perform the strength training activity based on an exercise history for the user. A variety of techniques may be used to evaluate or estimate this maximum capacity, and to adjust the raw intensity score accordingly. For example, adjusting the raw intensity score may include retrieving a scale factor from a load-repetition profile (such as the load-repetition profile illustrated in Fig. 5), which characterizes a set of repetitions relative to a user's maximum capacity over a range of loads and repetition counts. The intensity score for a set of repetitions may then be expressed as the product of the (motion-based) raw intensity score and the scaling factor indicative of a user's maximum capacity for performing repetitions of an exercise at a particular load. In another aspect, a scaling factor may be estimated or interpolated based on an observed or user-reported maximum load for a particular exercise, or an observed or reported maximum number of repetitions for a number of different loads. More generally, any technique suitable for quantitatively determining a user's maximum capacity for a type of a strength training activity, and/or scaling observed activities relating to the maximum capacity, may be used to scale raw intensity scores for a set of repetitions and provide an intensity score for the user. All such techniques are intended to fall within the scope of this disclosure provided they support a useful calculation of a musculoskeletal strain score as further described below.

It will also be appreciated that other techniques are known in the art for calculating an intensity and may be adapted for use with a wearable fitness monitor as described herein. For example, intensity may be evaluated based on the speed, linearity, and/or continuity of exercise-related motions, any of which may usefully be detected with motion sensors as described herein. For example, the amount of exertion can be identified based on how clean a path for an exercise motion is, or stated alternatively, based on the amount of noise in the motion relative to an expected trajectory and/or a historical trajectory for the user. A variety of linearity, continuity, and/or variation measures are known in mathematics, and may usefully be employed as estimators for intensity based on motion. In one aspect, the variability from an overall expected value, and/or the amount of motion outside of expected variances, may be used for an individual. In another aspect, localized measures of variation in directionality (e.g., quantity and magnitude of directional changes) or speed (e.g., quantity and magnitude of velocity changes) may be used to identify when an increased load results in a motion that is less smooth or continuous. In one aspect, the load may be measured across a major muscle group targeted by the exercise. For example, when a user is doing bicep curls, load on the bicep may be estimated based on any of the motion factors described above, as measured with a wrist-worn monitor. As another example, where a user is doing squats, a monitor located on the thigh or calf may be used to estimate load on various leg muscles. As a further example, where a user is doing pushups, dips, or another bodyweight exercise, a monitor on a torso of the user may be used to estimate intensity based on a range of motion of the portion of the torso. An intensity score may also be further contextualized as described herein using additional data, such as maximum or typical weights for a particular user, other related training for corresponding muscle groups, and so forth.

As shown in step 416, the method 400 may include updating a user profile. This may generally include updating the load-repetition profile, or any other user profile, as new data becomes available. In one aspect, this may include adding columns or rows to the profile. In another aspect, this may include updating other entries in the profile when, e.g., a current set of repetitions exceeds an expected maximum.

As shown in step 418, the method 400 may include calculating a volume for the strength training activity. As noted herein, volume generally refers to the total amount of work done during a workout (or other period of time). The volume may be individualized for a user based on two components: a maximum volume and an effective load. Thus, the method 400 may include, e.g., estimating a maximum volume or other predetermined threshold for the user while performing the strength training activity based on a history of user performance with the strength training activity. The maximum volume (or estimated maximum volume) may, for example, be indicative of an upper threshold for injury-free repetitions of the strength training activity by the user.

The method 400 may also include calculating an effective load for the user during the strength training activity. The effective load may, for example, be indicative of a relative portion of the maximum volume exerted by the user during the strength training activity based on one or more load parameters. The one or more load parameters may include at least a body weight of the user and an added weight for the strength training activity. For example, calculating the effective load may include calculating the effective load based on a user input of a body weight for the user, e.g., where the strength training activity is an activity such as pullups, pushups, or squats based in whole or in part on the user's body weight. Calculating the effective load may also be based on a user input of added weight for the strength training activity, e.g., where a user is using handheld weights, barbell weights, or weights on a strength training machine, or where the user is adding weight to another activity (such as pullups, pushups, or squats) to increase volume. In some instances, e.g., while using a strength training machine, body weight may be ignored, and/or some other load parameter may be included, such as arm length or leg length.

As shown in step 420, the method 400 may include calculating a musculoskeletal strain score. For example, this may include calculating a per repetition musculoskeletal strain for each one of the repetitions as a product of a first ratio of the effective load to the maximum volume and a second ratio of the raw intensity score to the maximum intensity, and then summing the per repetition musculoskeletal strain for all of the repetitions in the set to provide a musculoskeletal strain score for the strength training activity. In general, this may include calculating the musculoskeletal strain score on a personal computing device of the user coupled in a communicating relationship with the wearable monitor or calculating the musculoskeletal strain score on a remote server coupled in a communicating relationship with the wearable monitor, or some combination of these.

An MSK strain score may then be calculated using the following formula for each set of exercise:

$$\mathrm{MSK_{set}} = \sum_{i=1}^{M} MSK_{rep}^i = \sum_{i=1}^{M} \frac{V_{rep}^i}{V_{max}} \times \frac{I_{rep}^i}{I_{max}}$$

$$= \frac{1}{V_{max} \times I_{max}} \sum_{i=1}^{M} V_{rep}^i \times I_{rep}^i = \frac{V_{rep}}{V_{max} \times I_{max}} \sum_{i=1}^{M} I_{rep}^i$$

$$= V_{rep_{rel}} \sum_{i=1}^{M} I_{rep_{rel}}^i$$

where $M$ is the number of repetitions in a set, $V_{rep}^i$ and $I_{rep}^i$ are the volume and intensity of repetition $i$, respectively, $V_{max}$ and $I_{max}$ are the maximum possible volume and intensity values, respectively, and $rel$ refers to the relative volume and intensity of the repetitions. $V_{rep}^i$ has been replaced with $V_{rep}$ since it is a constant for the repetitions within a set. On the other hand, this is not the case for $I_{rep}$, which may be calculated for each repetition based on available motion data.

$$V_{rep_{rel}} = \frac{V_{rep}}{V_{max}}$$

$$I_{rep_{rel}}^i = \frac{I_{rep}^i}{I_{max}}$$

To accumulate MSK for multiple sets of an exercise:

$$\text{MSK}_{\text{exercise}} = \sum_{j=1}^N MSK_{set}^j = \sum_{j=1}^N V_{rep_{rel}}^j \sum_{i=1}^{M_j} I_{rep_{rel}}^i$$

where $N$ is the number of sets of the exercise.

Finally, for a session that is constituted of multiple exercises:

$$\text{MSK}_{\text{session}} = \sum_{k=1}^L MSK_{exercise}^k = \sum_{k=1}^L \sum_{j=1}^{N_k} V_{rep_{rel}}^j \sum_{i=1}^{M_j} I_{rep_{rel}}^i$$

$$= \sum_{k=1}^L \sum_{j=1}^{N_k} \sum_{i=1}^{M_j} \left( V_{rep_{rel}}^j \times I_{rep_{rel}}^i \right)$$

where L is the number of exercises in the session.

Volume and intensity maximums may be calibrated for an individual and can change as the individual's strength changes over time.

Using these, an MSK strain can be calculated for every repetition of an exercise. Aggregating the individual MSK strain scores can yield a workout-level score. At this level, MSK strains may be aggregated at the muscle group level, and/or at the whole-body level. When sensor data is available, intensity may be based on acceleration or velocity of motion. When sensor data is not available, effort can still be determined based on, e.g., previous workouts, demographic norms, and/or self-reported levels of exertion. Thus, systems and methods described herein may perform MSK scoring with motion data, without motion data, or some combination of these.

Raw MSK strain, as described herein, may be an unbounded linearly cumulative score. That is, the more effort that is put into an activity, or the more repetitions that are performed, the higher the score. To provide a bounded range for a user, these raw scores may be scaled to adjust the value based on a user's personal performance profile. In certain aspects, this can be achieved through a two-stage process that includes (1) exercise-specific normalization, and (2) performance normalization. The daily MSK strain for an individual may, for example, be transformed into a score on a scale of 0-21, or any other suitable range.

As shown in step 422, the method 400 may include taking an action based on the musculoskeletal (MSK) strain score. The MSK strain score provides a highly actionable metric for strength training activity, placing it in the context of a particular type of exercise, a demonstrated history of capacity, and an estimated injury limit for the user. In one aspect, taking an action may include displaying the musculoskeletal strain score for the strength training activity, or any other derived metric or analysis, to the user for viewing, e.g., on a user device.

In another aspect, taking an action may include refining a daily strain calculation (such as a cardiac strain calculation based on heart rate and/or heart rate variability) for the user based on the musculoskeletal strain score. For example, strain calculations based on cardiovascular activity (as described, e.g., in U.S. Pat. No. 11,185,292) may underestimate actual exertion during strength training activity. By determining an MSK load during strength training or other exercise, a total daily strain for a user may be updated to more accurately reflect strain due to muscle exertion as well as cardiovascular exertion.

In another aspect, taking an action may also or instead include calculating a plurality of musculoskeletal strain scores for each of a plurality of types of strength training activity in an exercise routine. These may be aggregated into a single MSK strain score for an entire workout routine made up of a number of separate strength training activities, and/or may be reported to the user as a single score or multiple scores.

Taking an action may also or instead include generating a coaching recommendation to the user based on the musculoskeletal strain score, and/or displaying the coaching recommendation to a user. This may include generating recommendations based on stated user objectives or fitness goals, e.g., by recommending increases where appropriate, or recommending decreases where there are signs of approaching the user's maximum volume or otherwise exceeding advisable training limits. In one aspect, the coaching recommendation may be a real time coaching recommendation presented to the user during a strength training activity. This may, for example, include a recommendation to increase volume (e.g., with additional repetitions or added weight), or a caution about approaching a maximum volume. In another aspect, the coaching recommendation may relate to a subsequent exercise activity by the user, such as a subsequent activity in a current workout, or the same activity (or a different activity) in a future workout. The coaching recommendation may also or instead include a recommendation about the timing for a next strength training activity.

According to the foregoing, there is also described herein a system for calculating a musculoskeletal strain score. The system may include a wearable fitness monitor and one or more processors. The wearable fitness monitor may be any wearable monitor described herein and may include one or more motion sensors. The one or more processors may, for example, include a processor on the wearable fitness monitor, a processor on a user device, a processor on a remote server, or some combination of these. The one or more processors may be configured by computer executable code stored in a non-transitory computer readable medium to perform the steps of : receiving motion data obtained from the one or more motion sensors during a strength training activity, identifying a type of the strength training activity, identifying a set of the strength training activity including one or more repetitions, for each one of the repetitions, calculating a raw intensity score indicative of musculoskeletal movement based on features of the motion data, and scaling the raw intensity score relative to a maximum intensity for performing the strength training activity by the user to obtain a per repetition user intensity score, the maximum intensity indicative of a capacity of the user to perform the strength training activity based on an exercise history for the user, for each one of the repetitions, calculating an individualization scale based on a ratio of an effective load for the user during the strength training activity and a predetermined load threshold for the user when performing the strength training activity, calculating a per repetition musculoskeletal strain for each one of the repetitions as a product of the per repetition user intensity score and the individualization scale, calculating a musculoskeletal strain score for the strength training activity by summing the per repetition musculoskeletal strain for all of the repetitions in the, and taking an action based on the musculoskeletal strain score.

In one aspect, taking an action may include transmitting the user-specific musculoskeletal strain score to a personal computing device associated with the user for display to the user. In another aspect, taking the action may include generating a coaching recommendation for the user.

In general, the methods described herein may include more or fewer steps, or variations to each of the steps described with reference to Fig. 4. For example, in one aspect, there is disclosed herein a method including receiving motion data from one or more motion sensors of a wearable monitor worn by a user during a strength training activity including a set of one or more repetitions; identifying a type of the strength training activity; determining a quantity of the repetitions in the set; for each one of the repetitions, calculating a raw intensity score indicative of musculoskeletal movement based on features of the motion data, and scaling the raw intensity score relative to a maximum intensity for performing the strength training activity by the user to obtain a per repetition user intensity score, the maximum intensity indicative of a capacity of the user to perform the strength training activity based on an exercise history for the user; for each one of the repetitions, calculating an individualization scale based on a ratio of an effective load for the user during the strength training activity and a predetermined load threshold for the user performing the strength training activity; calculating a per repetition musculoskeletal strain for each one of the repetitions as a product of the per repetition user intensity score and the individualization scale; calculating a musculoskeletal strain score for the strength training activity by summing the per repetition musculoskeletal strain for all of the repetitions in the; and taking an action based on the musculoskeletal strain score.

In another aspect, a method described herein includes receiving motion data from one or more motion sensors of a wearable fitness monitor worn by a user during a strength training activity; calculating an individualized musculoskeletal strain for the user during the strength training activity by adjusting an intensity associated with the motion data according to an exercise history for the user associated with the strength training activity, an effective load during the strength training activity, and a maximum volume for the user associated with the strength training activity; and taking an action based on the individualized musculoskeletal strain.

In another aspect, a method described herein includes receiving motion data from one or more motion sensors of a wearable fitness monitor worn by a user during a strength training activity; calculating a raw intensity score for a number of repetitions of the strength training activity based on the motion data; calculating an effective load for the strength training activity based on one or more load parameters including at least a body weight of the user and an added weight for the strength training activity; calculating an individualized musculoskeletal strain score based on a combination of the raw intensity score calculated from the motion data and the effective load calculated based on the one or more load parameters; and presenting information to the user based on the individualized musculoskeletal strain score.

Fig. 5 shows a load-repetition profile for scaling intensity of exercise repetitions. In general, this profile 500 may be used to scale the raw intensity score for a user based on workout history. In general, a maximum intensity value may be established at the user's one repetition maximum value (Load 10, Rep 1, in Fig. 5). Based on the maximum intensity for a single repetition, a scale can be developed for all intensity values for a given individual and exercise, e.g., by interpolating an effort percentage for scaling the calculated intensity based on the maximum possible value for the exercise. The profile 500 may also be adjusted as new repetitions to failure are observed, and new rows may be added as the user adds new numbers of repetitions or new loads. The numbers may more generally be adjusted, recalculated, re-interpolated, and the like as additional user data becomes available. In one aspect, if a user exceeds a predicted maximum, e.g., by performing one or more repetitions at a load exceeding the current maximum, the profile 500 may be adjusted before calculating the intensity, and the user's intensity for those repetitions may be calculated using the adjusted profile 500. This advantageously avoids calculating an intensity value exceeding the user's theoretical maximum.

In another aspect, an estimate for the intensity scale may initially be made based on, e.g., body weight or other factors in order to support calculations of intensity for the user before the profile 500 has been sufficiently populated.

Fig. 6 is a graph showing velocity over time measured during a bench press exercise. In general, the velocity may be derived from three-axis acceleration data, or other motion data obtained from a wearable monitor or some other source of motion data. While a set of bench presses are illustrated, it will be understood that any other exercise with a measurable repetition in motion may be similarly detected. As shown in the figure, five large peaks 602 in the velocity data indicate five repetitions of the exercise, and vertical lines 604 are added to indicate measurable landmarks for the end of each repetition. A variety of signal processing techniques may be used to identify such repetitions, including frequency domain techniques, time domain peak detection, and so forth. Any such technique suitable for identifying a periodic cycle of velocity measurements indicative of an exercise repetition may be used to automatically detect repetitions as described herein.

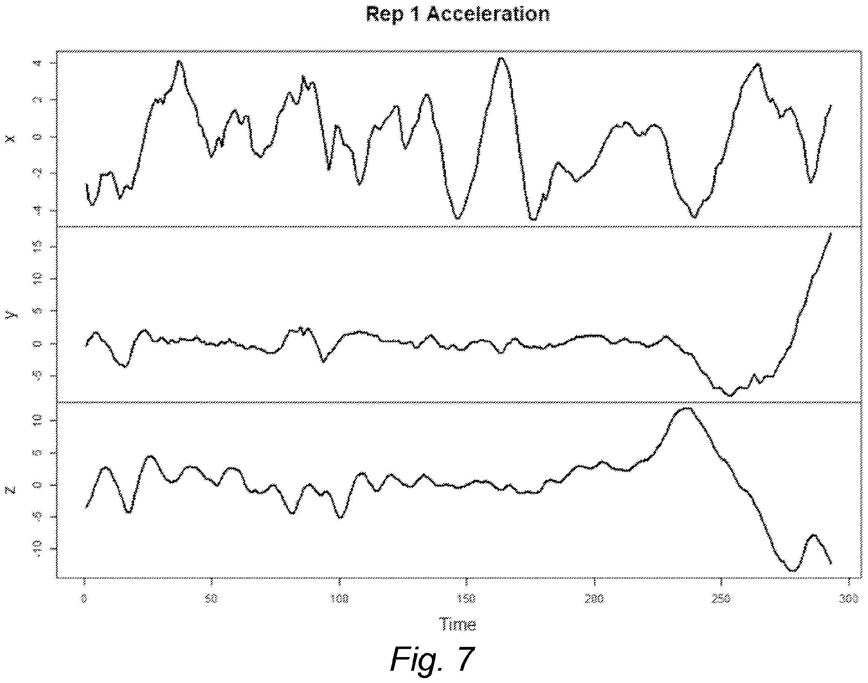

Fig. 7 shows three-axis acceleration data for one repetition of the bench press exercise. The data in Fig. 7 is fused data that has been processed to mitigate gravity-based acceleration artifacts. This data may be used, e.g., to calculate intensity scores, and/or to derive velocity data (such as that shown in Fig. 6) that can be used to identify individual repetitions in a set of a strength training activity.

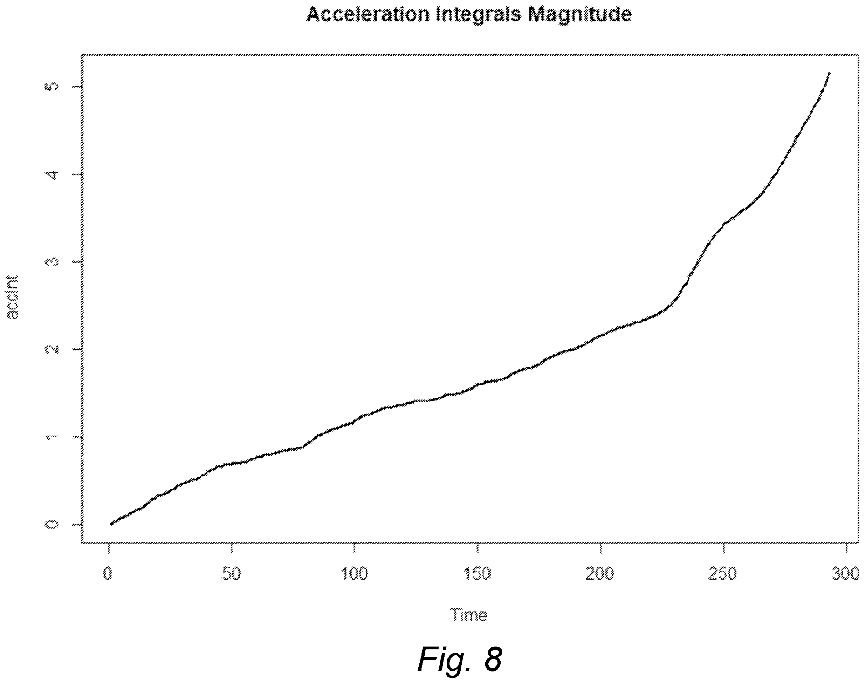

Fig. 8 shows an integral of normalized acceleration magnitudes for the data from Fig. 7. This may be used, e.g., to quantitatively evaluate intensity for an exercise, and then scaled according to the maximum intensity, maximum volume, and effective load as described herein to obtain an MSK score for a repetition of a strength training activity.

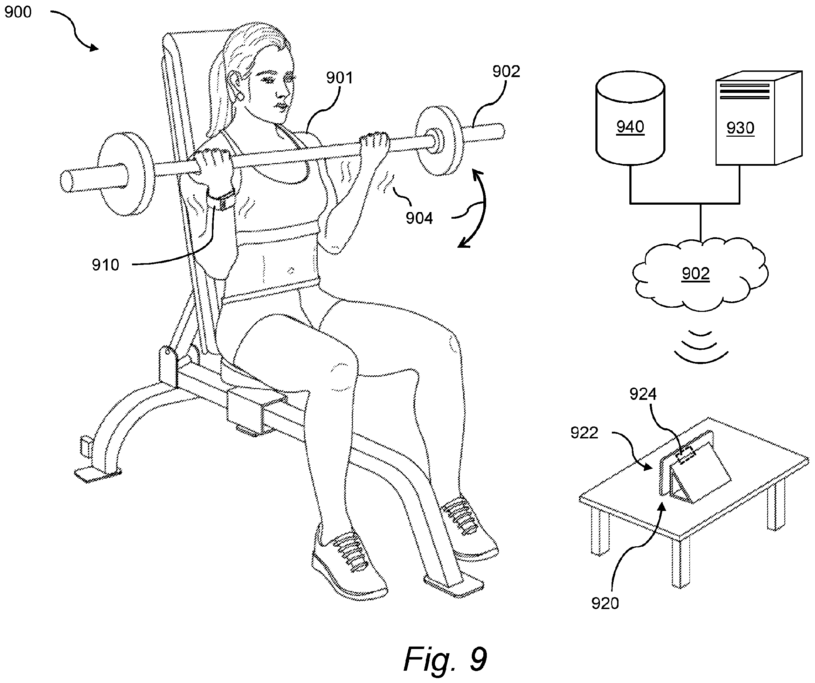

Fig. 9 shows a system for monitoring musculoskeletal (MSK) strain. The system 900 may include a user 901 wearing a physiological monitor 910, a user device 920 having a display 922 suitable for providing information to the user 901, a data network 902 interconnecting one or more participants of the system 900, a server 930, and a database 940. In general, Fig. 9 shows the user 901 performing an exercise-e.g., a weight training exercise such as a bicep curl with weights 902 in the form of a barbell with weighted plates thereon. As described herein, motion and/or physiological data sensed by the physiological monitor 910 may be used to calculate an MSK strain score for the user 901, which may be displayed to the user along with other related information via the user device 920 (e.g., during the exercise itself and/or subsequent to exercising).

The physiological monitor 910 may include a wrist-worn photoplethysmography device. The physiological monitor 910 may also or instead include monitors disposed in other locations on the body of the user 901 such as the bicep, the thigh, the calf, and so forth. In an aspect, the physiological monitor 910 includes at least one accelerometer, gyroscope, or the like for sensing motion and providing motion data for the user 901. In other aspects, accelerometer data or other motion sensor data is obtained from a source external to the physiological monitor 910.

The user 901 may be performing an exercise such as a strength training activity, where motion data (and/or physiological data) is provided to the user device 920 and/or the server 930 for analysis. In general, motion data captured by a motion sensor during an exercise can be analyzed to derive an MSK strain score as described herein. This score may be used, e.g., to provide coaching information to the user 901, e.g., for adjusting the exercise and/or providing other training recommendations. By way of example and not limitation, the user 901 is shown performing bicep curls with a barbell as the weights 902, where motion data from sensed motion 904 in this example can include three-axis acceleration data describing movement during repetitions of the exercise. This may include motion data acquired by the physiological monitor 910 as noted above, or by motion sensors in weights 902 (including the barbell or the weights added thereto), by a camera in the user device 920, or from any other suitable source. While a free weight exercise is depicted, it will be appreciated that the systems and methods described herein may be used to calculate MSK strain in various other strength training activities such as weightlifting exercises (e.g., using free weights and/or weight training machines), bodyweight or isometric exercises (e.g., pushups, sit ups, squats, burpees, dips, leg raises, and the like), cardiovascular exercises (e.g., walking, running, biking, swimming, elliptical exercises, circuit training, jump rope, sports participation and/or training, dancing, and the like), and so forth. Motion data may also be used for other coaching recommendations such as recommendations related to speed of repetitions, range of motion, form, and so forth. Thus, in one aspect there is described herein a system and method for providing coaching recommendations to a user engaging in a strength training activity based on motion sensed during the strength training activity. The recommendations may relate to one or more of a speed, a range of motion, and a form of the strength training activity.

The data network 902, the user device 920, the server 930, and the database 940 may be any as described herein. In general, the data network 902 may support communication among the participants in the system 901, such as where motion data and/or physiological data sensed by the physiological monitor 910 is provided to the server 930 or user device 920 for processing. Such data, and/or the results of analyses of that data, can be stored in the database 940, which can be a local or remote database as described herein. The database 940 may also or instead store user profiles, load-repetition profiles, and the like as described herein.

The display 922 of the user device 920 may include a graphical user interface provided on the display 922 and configured for presenting information to the user 901. The information 924 presented on the display 922 may include any output as described herein, including MSK strain scores 924, coaching recommendations, an exercise plan including a number of sequential strength training activities, repetitions completed in a particular set, and so forth.

In an example use case, the system 900 may receive information related to the exercise being performed by the user 901, such as the exercise type, set or repetition descriptions or metrics, training goals, added weights 902 or loads being used, information pertaining to the user 901 (e.g., height, weight, sex, etc.), and so forth. In some aspects, the information may include motion data from the physiological monitor 910 or other source(s).

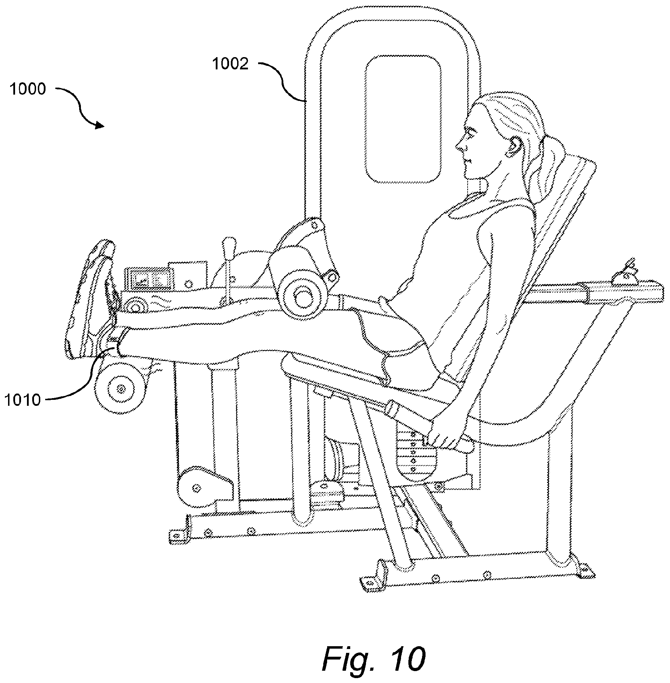

Fig. 10 shows a system for monitoring MSK strain. The system 1000 may include any of the features described herein, such as any features discussed above with respect to Fig. 9. As shown in Fig. 10, the system 1000 may include a weight training machine 1002. A physiological monitor 1010 may be disposed on a user's leg in order to detect motion of the leg during a leg strength training activity. In one aspect, the weight training machine 1002 may be a smart device capable of communicating data such as a current weight/load, number of repetitions, range of motion, and other data to the physiological monitor 1010 or other system resource for use in calculating an MSK strain score. The weight training machine 1002 may also or instead include a camera for capturing images that can be used to derive motion data.

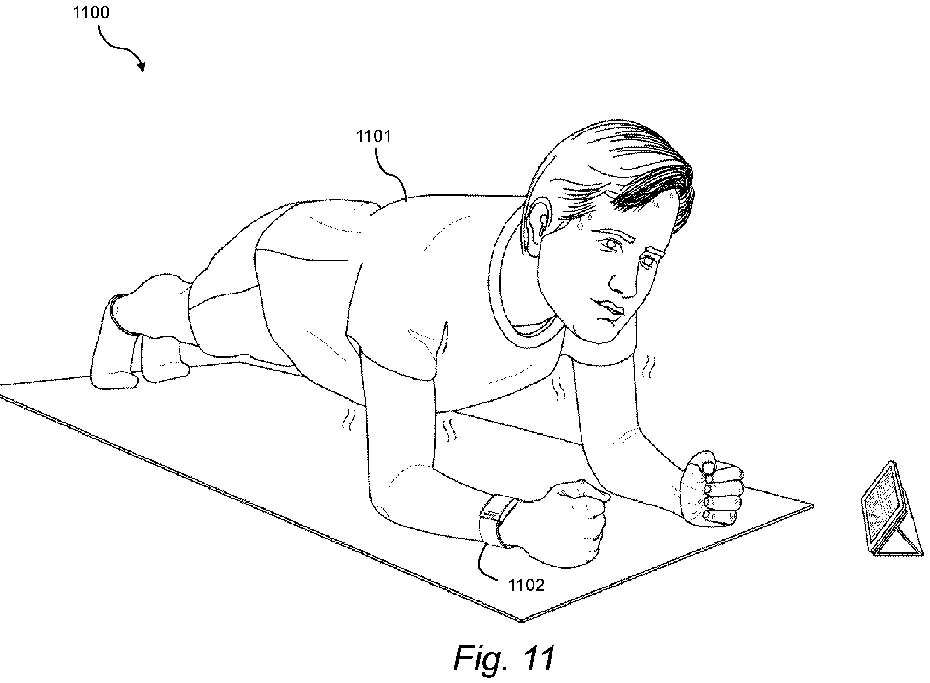

Fig. 11 shows a system for monitoring MSK strain. The system 1100 may include any of the features described herein. As illustrated, the user 1101 is performing an isometric exercise, more specifically a plank. In this type of exercise, certain adaptations to strain scoring may be used. For example, in a plank (or certain other isometric exercises) there are no literal repetitions. Rather, a repetition metric can be derived, e.g., based on the amount of time that the exercise is maintained. Thus, for example, the plank may be counted as one repetition every five seconds, such that a minute of performing the plank is equivalent to twelve repetitions. Similarly, there will be no periodic motion upon which to base an automatic detection of repetitions, but where exertion is high, there may be shaking of the shoulders, arms, or abdomen that may be detected by the wearable physiological monitor 1102 and used to calculate an intensity for the activity.

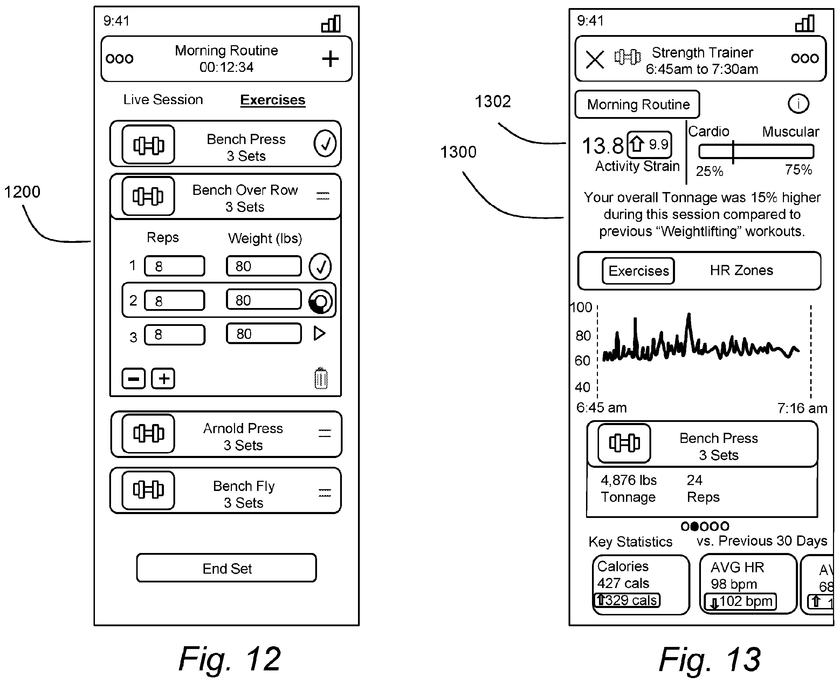

Fig. 12 shows a user interface for a strength training system with MSK strain scoring, e.g., using the systems and methods described herein. In one aspect, the user interface 1200 may include a number of controls to configure a workout routine, e.g., by specifying types of strength training activities, and repetitions and weights for each strength training activity as appropriate. Through this interface, a user may configure a workout routine by adding sets of exercises removing sets of exercises or specifying details for sets of exercises within the routine. A workout routine may then be saved for future use and used as a guide during a current workout.

Fig. 13 shows a user interface for a strength training system with MSK strain scoring, e.g., using the methods and systems described herein. In one aspect the user interface 1300 may display an MSK strain score 1302 that quantitatively summarizes an amount of musculoskeletal strain by the user for a current day, or any other suitable time period. The user interface 1300 may also display other useful information such as a current or most recent strength training activity, an amount of cardiovascular versus muscular strain for the day, coaching recommendations, and so forth. In this context, the MSK strain score 1302 may advantageously provide concise, quantitative, objective feedback to a user concerning recent strength training activities, as informed by identified activities and measurements of physical movements.

In the user interface 1300, the user may also track a current workout, modify a current workout (e.g., by changing weights, repetitions, or activities), review previous workouts, create new workouts, and so forth. The user may also review related information describing time, weights, exertion, cardiovascular strain, and the like. In another aspect, the user interface 1200 may provide interactive instructions on performing different types of exercises and may provide motion-based feedback on a user's form for a particular exercise.

The above systems, devices, methods, processes, and the like may be realized in hardware, software, or any combination of these suitable for the control, data acquisition, and data processing described herein. This includes realization in one or more microprocessors, microcontrollers, embedded microcontrollers, programmable digital signal processors or other programmable devices or processing circuitry, along with internal and/or external memory. This may also, or instead, include one or more application specific integrated circuits, programmable gate arrays, programmable array logic components, or any other device or devices that may be configured to process electronic signals. It will further be appreciated that a realization of the processes or devices described above may include computer-executable code created using a structured programming language such as C, an object oriented programming language such as C++, or any other high-level or low-level programming language (including assembly languages, hardware description languages, and database programming languages and technologies) that may be stored, compiled or interpreted to run on one of the above devices, as well as heterogeneous combinations of processors, processor architectures, or combinations of different hardware and software.

Thus, in one aspect, each method described above, and combinations thereof may be embodied in computer executable code that, when executing on one or more computing devices, performs the steps thereof. In another aspect, the methods may be embodied in systems that perform the steps thereof, and may be distributed across devices in a number of ways, or all of the functionality may be integrated into a dedicated, standalone device or other hardware. The code may be stored in a non-transitory fashion in a computer memory, which may be a memory from which the program executes (such as random access memory associated with a processor), or a storage device such as a disk drive, flash memory or any other optical, electromagnetic, magnetic, infrared, or other device or combination of devices. In another aspect, any of the systems and methods described above may be embodied in any suitable transmission or propagation medium carrying computer-executable code and/or any inputs or outputs from same. In another aspect, means for performing the steps associated with the processes described above may include any of the hardware and/or software described above. All such permutations and combinations are intended to fall within the scope of the present disclosure.

The method steps of the implementations described herein are intended to include any suitable method of causing such method steps to be performed, consistent with the patentability of the following claims, unless a different meaning is expressly provided or otherwise clear from the context. So, for example, performing the step of X includes any suitable method for causing another party such as a remote user, a remote processing resource (e.g., a server or cloud computer) or a machine to perform the step of X. Similarly, performing steps X, Y, and Z may include any method of directing or controlling any combination of such other individuals or resources to perform steps X, Y, and Z to obtain the benefit of such steps. Thus, method steps of the implementations described herein are intended to include any suitable method of causing one or more other parties or entities to perform the steps, consistent with the patentability of the following claims, unless a different meaning is expressly provided or otherwise clear from the context. Such parties or entities need not be under the direction or control of any other party or entity and need not be located within a particular jurisdiction.

It will be appreciated that the methods and systems described above are set forth by way of example and not of limitation. Numerous variations, additions, omissions, and other modifications will be apparent to one of ordinary skill in the art. In addition, the order or presentation of method steps in the description and drawings above is not intended to require this order of performing the recited steps unless a particular order is expressly required or otherwise clear from the context. Thus, while particular embodiments have been shown and described, it will be apparent to those skilled in the art that various changes and modifications in form and details may be made therein.

## Claims

1. A computer program product comprising computer executable code embodied in a non-transitory computer readable medium that, when executing on one or more computing devices, causes the one or more computing devices to perform the steps of:

   receiving (406) raw motion data from one or more motion sensors of a wearable fitness monitor worn by a user during a strength training activity including a set of one or more repetitions, the raw motion data including angular rotation data from a plurality of gyroscopes and linear acceleration data from a plurality of accelerometers;
   fusing (408) the raw motion data from the one or more motion sensors to mitigate gravitational artifacts, thereby providing motion data including three-axis acceleration data;
   identifying (410) a type of the strength training activity;
   determining (412) a quantity of the repetitions in the set based on variations in a magnitude of the three-axis acceleration data;
   for each one of the repetitions, calculating (414) a raw intensity score indicative of musculoskeletal movement based on changes in the three-axis acceleration data;
   for each one of the repetitions, determining a maximum intensity for performing the strength training activity by the user, the maximum intensity indicative of a capacity of the user to perform the strength training activity based on an exercise history for the user;
   estimating (418) a maximum volume for the user and the strength training activity based on a history of user performance with the strength training activity, wherein the maximum volume is indicative of an upper threshold for injury-free repetitions of the strength training activity by the user;
   calculating an effective load for the user during the strength training activity, the effective load indicative of a relative portion of the maximum volume exerted by the user during the strength training activity based on one or more load parameters including at least a body weight of the user and an added weight for the strength training activity;
   calculating (420) a per repetition musculoskeletal strain for each one of the repetitions as a product of a first ratio of the effective load to the maximum volume and a second ratio of the raw intensity score to the maximum intensity;
   summing (422) the per repetition musculoskeletal strain for all of the repetitions in the set to provide a musculoskeletal strain score for the strength training activity; and
   displaying the musculoskeletal strain score for the strength training activity to the user.

2. The computer program product of claim 1, further comprising code that causes the one or more computing devices to perform the step of generating a coaching recommendation to the user based on the musculoskeletal strain score.

3. The computer program product of claim 2, wherein the coaching recommendation is based at least in part on a fitness goal for the user.

4. The computer program product of any of claims 2 to 3, wherein the coaching recommendation is a real time coaching recommendation.

5. The computer program product of any of claims 2 to 3, wherein the coaching recommendation relates to a subsequent exercise activity by the user.

6. The computer program product of any of the preceding claims, wherein calculating the raw intensity score for one of the repetitions includes calculating a mean of a number of instantaneous intensity measurements for the one of the repetitions.

7. The computer program product of claim 6, wherein one or more of the number of instantaneous intensity measurements is calculated based on a mean of a ratio of discretely measured changes in a current acceleration to the current acceleration.

8. The computer program product of claim 6, wherein one or more of the number of instantaneous intensity measurements is calculated based on a ratio of a first mean of a current change in acceleration to a second mean of a current acceleration.

9. The computer program product of any of the preceding claims, further comprising code that causes the one or more computing devices to perform the step of creating a load-repetition profile for the user based on a history of the strength training activity by the user, the load-repetition profile indicating a capacity for repetitions by the user at one or more loads during the strength training activity.

10. The computer program product of claim 9, further comprising code that causes the one or more computing devices to perform the step of adding a row to the load-repetition profile when the user performs the strength training activity at a new load that is not included in the one or more loads in the load-repetition profile.

11. The computer program product of claim 9, further comprising code that causes the one or more computing devices to perform the step of updating the load-repetition profile when the user exceeds a number of repetitions for one of the loads in the load-repetition profile.

12. The computer program product of any of the preceding claims, further comprising code that receives a user input specifying the type of the strength training activity.

13. The computer program product of any of the preceding claims, further comprising code that causes the one or more computing devices to perform the step of identifying the type of the strength training activity based on the raw motion data.

14. The computer program product of any of the preceding claims, wherein determining the quantity of the repetitions in the set further includes determining the quantity based at least in part on user input.

15. The computer program product of any of the preceding claims, further comprising code that causes the one or more computing devices to perform the step of refining a daily strain calculation for the user based on the musculoskeletal strain score.

**Patentansprüche**

1. Computerprogrammprodukt, das einen in einem nicht flüchtigen, computerlesbaren Medium verkörperten, computerausführbaren Code umfasst, der bei Ausführung auf einer oder mehreren Rechenvorrichtungen bewirkt, dass die eine oder die mehreren Rechenvorrichtungen die Schritte durchführen von:

Empfangen (406) von Rohbewegungsdaten von einem oder mehreren Bewegungssensoren eines tragbaren Fitnessüberwachungsgeräts, der von einem Benutzer während einer Krafttrainingsaktivität getragen wird, die eine Reihe von einer oder mehreren Wiederholungen einschließt, wobei die Rohbewegungsdaten Winkelrotationsdaten von einer Vielzahl von Gyroskopen und lineare Beschleunigungsdaten von einer Vielzahl von Beschleunigungsmessern einschließen;
Zusammenführen (408) der Rohbewegungsdaten von dem einen oder den mehreren Bewegungssensoren, um Gravitationsartefakte zu mindern, wodurch Bewegungsdaten bereitgestellt werden, die dreiachsige Beschleunigungsdaten einschließen;

Identifizieren (410) einer Art der Krafttrainingsaktivität;

Bestimmen (412) einer Anzahl der Wiederholungen in der Reihe auf der Grundlage von Schwankungen in einer Größe der dreiachsigen Beschleunigungsdaten;

für jede der Wiederholungen, Berechnen (414) eines Rohintensitätswerts, der eine Bewegung des Bewegungsapparats auf der Grundlage von Änderungen in den dreiachsigen Beschleunigungsdaten angibt;

für jede der Wiederholungen, Bestimmen einer maximalen Intensität für die Durchführung der Krafttrainingsaktivität durch den Benutzer, wobei die maximale Intensität die Fähigkeit des Benutzers zur Durchführung der Krafttrainingsaktivität auf der Grundlage einer Trainingshistorie für den Benutzer angibt;

Schätzen (418) eines maximalen Volumens für den Benutzer und die Krafttrainingsaktivität auf der Grundlage einer Historie einer Benutzerleistung bei der Krafttrainingsaktivität, wobei das maximale Volumen einen oberen Schwellenwert für verletzungsfreie Wiederholungen der Krafttrainingsaktivität durch den Benutzer angibt;

Berechnen einer effektiven Belastung für den Benutzer während der Krafttrainingsaktivität, wobei die effektive Belastung einen relativen Anteil des maximalen Volumens angibt, das von dem Benutzer während der Krafttrainingsaktivität ausgeübt wird, auf der Grundlage von einem oder mehreren Belastungsparametern, einschließlich mindestens eines Körpergewichts des Benutzers und eines zusätzlichen Gewichts für die Krafttrainingsaktivität;

Berechnen (420) einer muskuloskelettalen Belastung pro Wiederholung für jede der Wiederholungen als ein Produkt eines ersten Verhältnisses der effektiven Belastung zu dem maximalen Volumen und eines zweiten Verhältnisses des Rohintensitätswerts zu der maximalen Intensität;

Summieren (422) der muskuloskelettalen Belastung pro Wiederholung für jede der Wiederholungen in der Reihe, um einen muskuloskelettalen Belastungswert für die Krafttrainingsaktivität bereitzustellen; und

Anzeigen des muskuloskelettalen Belastungswerts für die Krafttrainingsaktivität an den Benutzer.

2. Computerprogrammprodukt nach Anspruch 1, weiter umfassend Code, der bewirkt, dass die eine oder die mehreren Rechenvorrichtungen den Schritt des Erzeugens einer Trainingsempfehlung für den Benutzer auf der Grundlage des muskuloskelettalen Belastungswertes durchführen.

3. Computerprogrammprodukt nach Anspruch 2, wobei die Trainingsempfehlung mindestens teilweise auf einem Fitnessziel für den Benutzer basiert.

4. Computerprogrammprodukt nach einem der Ansprüche 2 bis 3, wobei die Trainingsempfehlung eine Echtzeit-Trainingsempfehlung ist.

5. Computerprogrammprodukt nach einem der Ansprüche 2 bis 3, wobei sich die Trainingsempfehlung auf eine nachfolgende Trainingsaktivität des Benutzers bezieht.

6. Computerprogrammprodukt nach einem der vorstehenden Ansprüche, wobei das Berechnen des Rohintensitätswerts für eine der Wiederholungen das Berechnen eines Mittelwerts einer Anzahl von Momentanintensitätsmessungen für die eine der Wiederholungen einschließt.

7. Computerprogrammprodukt nach Anspruch 6, wobei eine oder mehrere der Anzahl von Momentanintensitätsmessungen auf der Grundlage eines Mittelwerts eines Verhältnisses von diskret gemessenen Änderungen einer aktuellen Beschleunigung zu der aktuellen Beschleunigung berechnet werden.

8. Computerprogrammprodukt nach Anspruch 6, wobei eine oder mehrere der Anzahl von Momentanintensitätsmessungen auf der Grundlage eines Verhältnisses eines ersten Mittelwerts einer aktuellen Änderung der Beschleunigung zu einem zweiten Mittelwert einer aktuellen Beschleunigung berechnet werden.

9. Computerprogrammprodukt nach einem der vorstehenden Ansprüche, weiter umfassend Code, der bewirkt, dass die eine oder die mehreren Rechenvorrichtungen den Schritt des Erstellens eines Belastungswiederholungsprofils für den Benutzer auf der Grundlage einer Historie der Krafttrainingsaktivität durch den Benutzer durchführen, wobei das Belastungswiederholungsprofil eine Kapazität für Wiederholungen durch den Benutzer bei einer oder mehreren Belastungen während der Krafttrainingsaktivität angibt.

10. Computerprogrammprodukt nach Anspruch 9, weiter umfassend Code, der bewirkt, dass die eine oder die mehreren Rechenvorrichtungen den Schritt des Hinzufügens einer Zeile zu dem Belastungswiederholungsprofil durchführen, wenn der Benutzer die Krafttrainingsaktivität mit einer neuen Belastung durchführt, die nicht in der einen oder den mehreren Belastungen in dem Belastungswiederholungsprofil eingeschlossen ist.

11. Computerprogrammprodukt nach Anspruch 9, weiter umfassend Code, der bewirkt, dass die eine oder die mehreren Rechenvorrichtungen den Schritt des Aktualisierens des Belastungswiederholungsprofils durchführen, wenn der Benutzer eine Anzahl von Wiederholungen für eine der Belastungen in dem Belastungswiederholungsprofil überschreitet.

12. Computerprogrammprodukt nach einem der vorstehenden Ansprüche, weiter umfassend Code, der eine Benutzereingabe empfängt, die die Art der Krafttrainingsaktivität spezifiziert.

13. Computerprogrammprodukt nach einem der vorstehenden Ansprüche, weiter umfassend Code, der bewirkt, dass die eine oder die mehreren Rechenvorrichtungen den Schritt des Identifizierens der Art der Krafttrainingsaktivität auf der Grundlage der Rohbewegungsdaten durchführen.

14. Computerprogrammprodukt nach einem der vorstehenden Ansprüche, wobei das Bestimmen der Anzahl der Wiederholungen in der Reihe weiter das Bestimmen der Anzahl mindestens teilweise auf der Grundlage einer Benutzereingabe einschließt.

15. Computerprogrammprodukt nach einem der vorstehenden Ansprüche, weiter umfassend Code, der bewirkt, dass die eine oder die mehreren Rechenvorrichtungen den Schritt des Verfeinerns einer täglichen Belastungsberechnung für den Benutzer auf der Grundlage des muskuloskelettalen Belastungswerts durchführen.

**Revendications**

1. Produit de programme d'ordinateur comprenant un code exécutable par ordinateur incorporé dans un support non transitoire lisible par ordinateur qui, lorsque s'exécutant sur un ou plusieurs dispositifs informatiques, amène le un ou les plusieurs dispositifs informatiques à réaliser les étapes consistant à :

recevoir (406) des données de mouvement brutes provenant d'un ou de plusieurs capteurs de mouvement d'un dispositif de surveillance de la condition physique portable porté par un utilisateur pendant une activité d'entraînement de la force incluant un ensemble d'une ou de plusieurs répétitions, les données de mouvement brutes incluant des données de rotation angulaire provenant d'une pluralité de gyroscopes et des données d'accélération linéaire provenant d'une pluralité d'accéléromètres ;
fusionner (408) les données de mouvement brutes provenant du un ou des plusieurs capteurs de mouvement pour atténuer des artéfacts gravitationnels, ce qui permet de fournir des données de mouvement incluant des données d'accélération sur trois axes ;
identifier (410) un type de l'activité d'entraînement de la force ;
déterminer (412) une quantité des répétitions dans l'ensemble sur la base de variations d'une amplitude des données d'accélération sur trois axes ;
pour chacune des répétitions, calculer (414) un score d'intensité brut indicatif d'un mouvement musculo-squelettique sur la base de changements des données d'accélération sur trois axes ;
pour chacune des répétitions, déterminer une intensité maximale permettant de réaliser l'activité d'entraînement de la force par l'utilisateur, l'intensité maximale indicative d'une capacité de l'utilisateur à réaliser l'activité d'entraînement de la force sur la base d'un historique d'exercice pour l'utilisateur ;
estimer (418) un volume maximal pour l'utilisateur et l'activité d'entraînement de la force sur la base d'un historique de performances d'utilisateur avec l'activité d'entraînement de la force, dans lequel le volume maximal est indicatif d'un seuil supérieur pour des répétitions sans blessure de l'activité d'entraînement de la force par l'utilisateur ;
calculer une charge effective pour l'utilisateur pendant l'activité d'entraînement de la force, la charge effective étant indicative d'une partie relative du volume maximal exercé par l'utilisateur pendant l'activité d'entraînement de la force sur la base d'un ou de plusieurs paramètres de charge incluant au moins un poids de corps de l'utilisateur et un poids ajouté pour l'activité d'entraînement de la force ;
calculer (420) une tension musculo-squelettique par répétition pour chacune des répétitions en tant qu'un produit d'un premier rapport de la charge effective au volume maximal et d'un second rapport du score d'intensité brut à l'intensité maximale ;
faire la somme (422) de la tension musculo-squelettique par répétition pour toutes les répétitions dans l'ensemble de manière à fournir un score de tension musculo-squelettique pour l'activité d'entraînement de la force ; et

afficher le score de tension musculo-squelettique pour l'activité d'entraînement de la force à l'utilisateur.

2. Produit de programme d'ordinateur selon la revendication 1, comprenant en outre un code qui amène le un ou les plusieurs dispositifs informatiques à réaliser l'étape consistant à générer une recommandation d'accompagnement pour l'utilisateur sur la base du score de tension musculo-squelettique.

3. Produit de programme d'ordinateur selon la revendication 2, dans lequel la recommandation d'accompagnement est basée au moins en partie sur un objectif de condition physique de l'utilisateur.

4. Produit de programme d'ordinateur selon l'une quelconque des revendications 2 et 3, dans lequel la recommandation d'accompagnement est une recommandation d'accompagnement en temps réel.

5. Produit de programme d'ordinateur selon l'une quelconque des revendications 2 et 3, dans lequel la recommandation d'accompagnement est relative à une activité d'exercice ultérieure par l'utilisateur.

6. Produit de programme d'ordinateur selon l'une quelconque des revendications précédentes, dans lequel le calcul du score d'intensité brut pour l'une des répétitions inclut le calcul d'une moyenne d'un nombre de mesures d'intensité instantanées pour l'une des répétitions.

7. Produit de programme d'ordinateur selon la revendication 6, dans lequel un ou plusieurs du nombre de mesures d'intensité instantanées est ou sont calculés sur la base d'une moyenne d'un rapport de changements mesurés de manière discrète d'une accélération actuelle à l'accélération actuelle.

8. Produit de programme d'ordinateur selon la revendication 6, dans lequel un ou plusieurs du nombre de mesures d'intensité instantanées est ou sont calculés sur la base d'un rapport d'une première moyenne d'un changement actuel d'accélération à une seconde moyenne d'une accélération actuelle.

9. Produit de programme d'ordinateur selon l'une quelconque des revendications précédentes, comprenant en outre un code qui amène le un ou les plusieurs dispositifs informatiques à réaliser l'étape consistant à créer un profil de répétition de charge pour l'utilisateur sur la base d'un historique de l'activité d'entraînement de la force par l'utilisateur, le profil de répétition de charge indiquant une capacité pour des répétitions par l'utilisateur au niveau d'une ou de plusieurs charges pendant l'activité d'entraînement de la force.

10. Produit de programme d'ordinateur selon la revendication 9, comprenant en outre un code qui amène le un ou les plusieurs dispositifs informatiques à réaliser l'étape consistant à ajouter une ligne au profil de répétition de charge lorsque l'utilisateur réalise l'activité d'entraînement de la force au niveau d'une nouvelle charge qui n'est pas incluse dans la une ou les plusieurs charges dans le profil de répétition de charge.

11. Produit de programme d'ordinateur selon la revendication 9, comprenant en outre un code qui amène le un ou les plusieurs dispositifs informatiques à réaliser l'étape consistant à actualiser le profil de répétition de charge lorsque l'utilisateur dépasse un nombre de répétitions pour l'une des charges dans le profil de répétition de charge.

12. Produit de programme d'ordinateur selon l'une quelconque des revendications précédentes, comprenant en outre un code qui reçoit une entrée d'utilisateur spécifiant le type de l'activité d'entraînement de la force.

13. Produit de programme d'ordinateur selon l'une quelconque des revendications précédentes, comprenant en outre un code qui amène le un ou les plusieurs dispositifs informatiques à réaliser l'étape consistant à identifier le type de l'activité d'entraînement de la force sur la base des données de mouvement brutes.

14. Produit de programme d'ordinateur selon l'une quelconque des revendications précédentes, dans lequel la détermination de la quantité des répétitions dans l'ensemble inclut en outre la détermination de la quantité sur la base au moins en partie d'entrées d'utilisateur.

15. Produit de programme d'ordinateur selon l'une quelconque des revendications précédentes, comprenant en outre un code qui amène le un ou les plusieurs dispositifs informatiques à réaliser l'étape consistant à affiner un calcul de tension quotidienne pour l'utilisateur sur la base du score de tension musculo-squelettique.

Fig. 1

*Fig. 2*

*Fig. 3*

EP 4 358 838 B1

*Fig. 4*

RECEIVE USER DATA
404

RECEIVE MOTION DATA
406

PROCESS MOTION DATA
408

IDENTIFY ACTIVITY
410

DETERMINE QUANTITY OF REPETITIONS
412

CALCULATE INTENSITY
414

UPDATE USER PROFILE
416

CALCULATE VOLUME
418

CALCULATE MSK STRAIN
420

TAKE ACTION BASED ON MSK STRAIN
422

400

500

| | Rep 1 | Rep 2 | Rep 3 | Rep 4 | Rep 5 | Rep 6 | Rep 6 | Rep 8 | Rep 9 | Rep 10 |
|---|---|---|---|---|---|---|---|---|---|---|
| Load 1 | 1 | 1 | 2 | 2 | 3 | 3 | 3 | 4 | 4 | 5 |
| Load 2 | 2 | 3 | 3 | 4 | 5 | 5 | 6 | 6 | 7 | 8 |
| Load 3 | 3 | 4 | 4 | 5 | 6 | 6 | 7 | 7 | 8 | 8 |
| Load 4 | 4 | 4 | 5 | 6 | 6 | 7 | 7 | 8 | 9 | 9 |
| Load 5 | 6 | 7 | 8 | 9 | 10 | 12 | | | | |
| Load 6 | 7 | 8 | 9 | 11 | 13 | | | | | |
| Load 7 | 9 | 10 | 11 | 13 | | | | | | |
| Load 8 | 10 | 11 | 13 | | | | | | | |
| Load 9 | 10 | 13 | | | | | | | | |
| Load 10 | 13 | | | | | | | | | |

*Fig. 5*

*Fig. 6*

Rep 1 Acceleration

Fig. 7

EP 4 358 838 B1

*Fig. 8*

Fig. 9

1002

1000

1010

Fig. 10

*Fig. 11*

9:41

X ⬛ Strength Trainer
6:45am to 7:30am

Morning Routine

13.8 ⬆9.9
Activity Strain

Cardio 25% | Muscular 75%

Your overall Tonnage was 15% higher during this session compared to previous "Weightlifting" workouts.

Exercises | HR Zones

100
80
60
40

6:45 am — 7:16 am

Bench Press
3 Sets

4,876 lbs
Tonnage

24
Reps

Key Statistics — vs. Previous 30 Days

Calories
427 cals
⬇329 cals

AVG HR
98 bpm
⬇102 bpm

AV
68

1302

1300

*Fig. 13*

9:41 +

Morning Routine
00:12:34

Live Session   Exercises

Bench Press
3 Sets

Bench Over Row
3 Sets

Weight (lbs)   Reps

80   1   8
80   2   8
80   3   8

+
—

Arnold Press
3 Sets

Bench Fly
3 Sets

End Set

1200

*Fig. 12*

**REFERENCES CITED IN THE DESCRIPTION**

**Patent documents cited in the description**

- US 63395244
- WO 2021023357 A1
- WO 2015048884 A1
- US 2021128979 A1
- US 2018133551 A1
- US 11185292 B